# EUROPEAN PATENT APPLICATION

(11) **EP 4 407 315 A1**
(43) Date of publication of application: **31.07.2024**
(21) Application number: 23382063.8
(22) Date of filing: 26.01.2023
(51) Int. Cl.: G01N 33/50, A61K 39/395, A61K 48/00, A61P 19/08, C12Q 1/6883, G01N 33/68

(54) **DIAGNOSIS AND TREATMENT OF NEUROGENIC HETEROTOPIC OSSIFICATION**

(71) Applicant: Servizo Galego de Saude, 15703 Santiago de Compostela (ES); Fundación Instituto de Investigación Sanitaria de Santiago de Compostela, 15706 Santiago de Compostela, A Coruña (ES); Universidade de Santiago de Compostela, 15782 Santiago de Compostela (ES)
(72) Inventor: GÓMEZ BAHAMONDE, Rodolfo, 15706 SANTIAGO DE COMPOSTELA (ES); LÓPEZ FAGÚNDEZ, Miriam, 15706 SANTIAGO DE COMPOSTELA (ES); FRANCO TREPAT, Eloi, 15706 SANTIAGO DE COMPOSTELA (ES); ALONSO PÉREZ, Ana, 15706 SANTIAGO DE COMPOSTELA (ES); GUILLÁN FRESCO, María, 15706 SANTIAGO DE COMPOSTELA (ES); PAZOS PÉREZ, Andrés, 15706 SANTIAGO DE COMPOSTELA (ES); JORGE MORA, Alberto Agustín, 15706 SANTIAGO DE COMPOSTELA (ES)
(74) Representative: Hoffmann Eitle

(57) **Abstract**

The present invention is focused on an *in vitro* method for the diagnosis or prognosis of Neurogenic Heterotopic Ossification (NHO). Moreover, the present invention also refers to a therapeutic strategy for treating NHO.

## Description

### FIELD OF THE INVENTION

The present invention refers to the medical filed. Particularly, the present invention is focused on an *in vitro* method for the diagnosis or prognosis of Neurogenic Heterotopic Ossification (NHO). Moreover, the present invention also refers to a therapeutic strategy for treating NHO.

### STATE OF THE ART

The term Heterotopic Ossification (HO) refers to the presence of mature lamellar bone in extra-skeletal soft tissue. The bone growth can occur through endochondral or intramembranous ossification mechanisms.

Clinically, there are no pathognomonic signs to establish an early diagnosis of this disease. Thus, its diagnosis is mainly based on non-specific signs and symptoms.

Currently, the etiopathology of this disease remains unclear. However, some risk factors contribute to its development. Among them, neurogenic insults, fractures, or joint replacements should be highlighted. Nonetheless, it has been described that for ectopic bone formation, a triggering factor, osteoblastic precursors, and an environment that promotes osteoblastogenesis are necessary.

According to its etiology, this disease can be classified as genetic or acquired HO. Genetic variants (Fibrodysplasia Ossificans Progressiva and Progressive Osseous Heteroplasia) are rare diseases and are considered the most severe HO manifestations. Regarding non-hereditary forms, they represent the most frequent HO variant. The acquired HO can develop after traumatism or a neurogenic insult.

The NHO is one of the most frequent complications of suffering an injury in the Central Nervous System (CNS). According to this, it has been demonstrated that its incidence increases after concomitant CNS damage, such as a traumatic brain injury (TBI) or spinal cord injury, and a peripheral injury, such as a long bone fracture (LBF).

The pathophysiology of NHO remains unknown. Nonetheless, it has been hypothesized that the humoral neuroimmunological factors released by the brain could play a critical role in developing this disease. After a TBI, the blood-brain barrier (BBB) is compromised, and some neurogenic factors leak from the brain to distal tissues. At the same time, distal tissues located close to the fracture secrete other factors to promote bone healing. Thus, it was suggested that the convergence of these two factors could potentiate abnormal bone growth. Likewise, studies pointed to osteoblast precursors and circulating cells with osteoforming capacity as potential factors to accelerate ectopic bone formation.

Recently, the Peripheral Nervous System (PNS) has also been postulated as a promoter of heterotopic bone. Specifically, studies linked the disruption of the blood-nerve barrier (BNB) with an increased in NHO incidence. Additionally, the presence of osteoblast precursors in nerves has also been suggested.

Even though there is a high incidence of this pathology, the therapies used for its management have low-effective rates. Moreover, some of them are highly invasive and associated with side effects and complications.

So, there is an unmet medical need of finding effective methods for the diagnosis, prognosis and treatment of NHO. The present invention is focused on solving this problem and a new strategy is herein provided.

### DESCRIPTION OF THE INVENTION

### Brief description of the invention

The present invention refers to an *in vitro* method for the diagnosis or prognosis of NHO and also to a therapeutic strategy for treating NHO.

Initially, the inventors of the present invention developed an *in vitro* Traumatic Brain Injury (TBI) model to investigate the effects of the unknown released factors on osteoblastogenesis, and adipogenesis. To do this, astrocytes and osteoblasts were co-cultured. Then, the RNA and proteins were extracted to analyse the effects of the co-culture on osteoblasts. The obtained results revealed how the co-culture of astrocytes and osteoblasts increased the expression of two of the main bone markers related to HO: SPP1 and BMP2. Considering these results, the inventors of the present invention investigated if the observed effects were due to the effect of astrocytes on osteoblasts, or vice versa. Thus, the co-culture was deconstructed. Astrocytes were cultured and let them conditioning the medium for 72 hours. After that, the astrocyte supernatant was added to the osteoblasts. The results previously observed in SPP1 and BMP2 bone markers were still preserved, which suggests that astrocytes secrete some factors that are able to induce the expression of bone related genes in osteoblasts. Considering that the effects were similar in the co-culture and in the astrocyte conditioned medium (ACM) model, the rest of the experiments were carried out with the ACM at day 3, and study other bone related bone markers. Thus, ACM increased the expression of bone related markers such as CD44 (the receptor of osteopontin), and LIF on osteoblasts. Moreover, ACM induced the expression of AXIN2, and decreased the expression of SOST and DKK1, which suggest the activation of the WNT pathway (the main bone anabolic pathway). Interestingly, the ratio RANKL/OPG was decreased by the ACM, which indicates a modulation of bone remodelling towards bone anabolism. Additionally, it was also found cell morphological changes induced by the ACM. Thus, an actin and tubulin staining was performed. Surprisingly, these morphologic changes resembled osteocytes morphology. Considering these findings, it was decided to study the expression of osteogenic markers. Consistent with the morphogenic changes, ACM increased the expression of PDPN, which is the earliest osteocyte marker. Nonetheless, the other marker was not upregulated by the ACM. These results support that ACM may accelerate bone formation process by accelerating the differentiation of osteoblasts into osteocytes. With these results it is demonstrated that ACM is able to increase bone anabolism. It is well known that certain grade of inflammation is necessary to bone healing. Thus, the effect of ACM on osteoblast inflammatory responses was also investigated. In this context, the expression key inflammatory markers (IL6, VCAM, and CCL2) were studied. The results showed a significantly increase of these three genes by the ACM. Thus, ACM not only could increase bone anabolism, but also inflammation on osteoblasts. Considering that astrocyte medium could induce inflammation on osteoblasts *per se,* it was decided to stimulate osteoblasts with an inflammatory stimulus like IL1B, to mimic the inflammatory environment associated to TBI and the LBF. Moreover, osteoblasts were stimulated after 72 hours with the ACM, and left it with the IL1B for another 72 hours. Interestingly, it was observed that IL1B synergised with the ACM, and increased the expression of the 3 inflammatory genes studied. Surprisingly, it was also found that IL1B stimulation increased some bone related markers such as BMP2 and LIF in osteoblasts stimulated with the ACM. This further supports the link between inflammation and bone anabolism. Considering that osteoblastogenesis and adipogenesis are balanced processes and antithetic, it was decided to explore the effect of the ACM on MSC (mesenchymal stem cells) differentiated to adipocytes. To do this, astrocytes conditioned the medium for 72 hours as previously described. Afterwards, their supernatant was centrifuged and filtered to obtain the ACM pellet. Finally, it was resuspended with the adipogenic medium to differentiate the MSC to adipocytes for 7 days. The obtained results showed a significant decrease of three of the four adipogenic markers studied (FABP4, PPARG, and ADIPOQ). To confirm this, the lipid droplets were stained, and the results showed the capacity of ACM to totally blunt the accumulation of lipid droplets. As MSC were not differentiated into adipocytes, we decided to study some bone markers in the adipogenesis model. The results showed an increase of SPP1 and RUNX2 in MSC differentiated to adipocytes. So, ACM not only suppressed adipogenesis but also promoted the expression of bone-related markers in MSC when they were differentiated into adipocytes. Finally, it was also discovered that MSC cells differentiated with the ACM proliferate more than those treated with the differentiated medium (DM). To validate this, a MTT was performed for 7 days, and the results were in line with what we saw before: ACM exerted a proliferative effect on MSC when they were differentiated into adipocytes. Altogether, these results suggest that the ACM alters the osteoblast/adipogenesis balance by promoting osteoblastic precursors. To better identify the responsible factors of the previous results, it was decided to run a proteomics experiment of the supernatants. These proteins were analysed by a quantitative (SWATH) and qualitative (DDA) methods. Among the proteins, one of them was highly expressed in the ACM: the TGFBi (Unitprot Q15582). Concretely, this protein was increased 14 times more in the ACM than in the DM. To validate the proteomic results, a western blot was performed. The results evidenced the TGFBi protein expression was higher in osteoblasts treated with the ACM, when compared with the differentiated medium. A RT-PCR was also performed on osteoblasts to elucidate the expression levels of TGFBi in these cells. The results showed that ACM was able to increase TGFBi expression levels in osteoblast. This suggests that osteoblasts can also amplify the levels of this factor. To figure out the contribution of TGFBi on the ACM effect on osteoblasts, they were stimulated with TGFBi recombinant for 3 days. The results showed a partial recapitulation of the results obtained when osteoblasts were treated with the ACM, regarding the expression of SPP1, BMP2, VCAM and CCL2. Regarding the effect of TGFBi on adipogenesis, the effect in one of the adipogenic markers was recapitulated. Specifically, TGFBi stimulation significantly decreased the expression of PPARG, a master regulator of the adipogenesis. This could be attributed to the TGFBi concentration we used, as well as the missing interactions of TGFBi with other proteins in the ACM. Additionally, in MSC treated with TGFBi, differences on cell proliferation were observed, similar to those seen when cells were treated with the ACM. This appreciation was validated by quantifying the RNA, as well as by counting the number of cells. To sum up, TGFBi partially recapitulated the effects of ACM on osteoblastogenesis, adipogenesis, and cell proliferation. These results highlight TGFBi as a potential biomarker as well as therapeutic target for HO. However, it was necessary to know if patients at risk of developing this disease also present this protein. Thus, the serum of patients with high risk of developing NHO (TBI+LBF) was collected, with medium and low risk of developing the disease (TBI and LBF, respectively) and at non-risk of developing NHO (controls). Blood samples were collected at day 0, 1, 3, and 7 post-trauma. Only one sample was collected in the control group. It is believed that double trauma patients present higher anabolic circulating factors. Thus, the serum proteomic profiles were analysed and correlated with their effects on bone metabolism. The results evidenced a higher number of correlations in the double trauma group (TBI+LBF), when compared to the other groups. To further characterise the double trauma group, a pathway enrichment analysis was performed. Consistent with the idea assessed in the *in vitro* model, an inflammatory environment was identified in the serum of these patients. Interestingly, a significant enrichment of TGFB signalling was identified, which is consistent with the literature, and also with the *in vitro* findings of the induction of TGFBi. Finally, the expression of TGFBi in serum patients was assessed by western blot. The results showed that patients with double trauma presented higher levels of TGFBi, reaching its peak at day 3 (which is in concordance with our in vitro TBI model). Altogether, these data point TGFBi as a potential biomarker and therapeutic target for NHO. In the context of NHO, recent studies also suggest the contribution of peripheral nervous system damage on the formation of ectopic bone. To elucidate the role of nerves on bone anabolism, we cocultured nerve explants (NE), as well as the outgrowth nerve cells (Nerve cells) with osteoblasts. Firstly, it was decided to characterise the secretome profile by proteomics. Results elucidated a higher TGFBi expression on NE conditioned medium when compared with the differentiated medium. Finally, these results were validated by western blot. Then, the gene expression of the main bone markers was studied on osteoblasts co-cultured with the NE. The results showed a significant increase of SPP1, RUNX2, BMP2, LIF, some bone remodelling and inflammatory markers, and it was also demonstrated that the nerves can induce the expression of TGFBi on osteoblasts. In view of these results, a similar profile to the one detected before with the astrocytes was identified. Interestingly, when the TGFBi expression of healthy nerves and congenital HO nerves were compared, it was seen that this protein is highly produced in HO samples. Following the same method, the effect of nerve cells on osteoblastogenesis was tested. Although the effect was not as strong as the co-culture with the NE, nerve cells increased the expression of SPP1, VCAM, and TGFBi. One of the reasons of the lower effect observed could be due to that the NE contains a higher number of cells than the number of cells we cultured in this experiment. However, this data are really interesting because these cells can migrate and target different tissues, in which they can reproduce part of the phenotype induced by the nerves. Finally, the effect of the NE on adipogenesis was tested. The results showed a decrease in three of the adipogenic markers, as well as an increase of SPP1, and TGFBi in MSC differentiated to adipocytes. Moreover, nerves were also able to increase the expression of the inflammatory markers IL6, VCAM, and CCL2. Altogether, this data suggest that central and peripheral nervous systems are able to modulate the osteoblastogenesis-adipogenesis balance, by potentiating the osteoblastogenesis. Moreover, it is herein demonstrated that TGFBi could play a crucial role in bone formation, trough the potentiation of the osteoblastogenesis and inhibition of the adipogenesis. Therefore, TGFBi could be a potential biomarker and used as a therapeutic target for NHO.

So, the first embodiment of the present invention refers to an *in vitro* method for the diagnosis or prognosis of NHO which comprises assessing the expression level of TGFBi in a biological sample obtained from the subject, wherein the determination of an increase of the expression level of TGFBi with respect to a pre-established threshold value of the expression level measured in healthy control subjects, is an indication that the subject may be suffering from NHO or has a poor prognosis.

In a preferred embodiment, the biological sample is selected from: blood, plasma, serum, tissue biopsy from, for instance, peripheral nerves comprising saphenous, posterior tibial and sciatic nerves or synovial fluid.

The second embodiment of the present invention refers to the *in vitro* use of TGFBi, or of a kit comprising reagents for assessing the expression level of TGFBi, for the diagnosis or prognosis of NHO.

The third embodiment of the present invention refers to TGFBi inhibitors, for instance selected from siRNA or TGFBi neutralizing antibodies, for use in the treatment of NHO.

In a preferred embodiment the siRNA is:
- hs.Ri.TGFBi 13.1:
   SEQ ID NO: 1 > 5-GUGGCAAAUCAACAGUCAUCAGCTA-3'
   SEQ ID NO: 2 > 5-UAGCUGAUGACUGUUGAUUUGCCACAG-3'
- hs. Ri. TGFBi 13.2:
   SEQ ID NO: 3 > 5-GUUUUCAAAACCAAGUAUCACACTT-3'
   SEQ ID NO: 4 > 5-AAGUGUGAUACUUGGUUUUGAAAACAU-3'
- hs. Ri. TGFBi 13.3:
   SEQ ID NO: 5 > 5-CUACAUUGAUGAGCUACUCAUCCCA-3'
   SEQ ID NO: 6 > 5-UGGGAUGAGUAGCUCAUCAAUGUAGUG-3'

In a preferred embodiment the TGFBi neutralizing antibody is:
- Immunogen Catalog Number: Ag0241.
- GenBank Accession Number: BC000097.
- Gene ID (NCBI): 7045.
- Proteintech. Catalog Number: 10188-1-AP

In a preferred embodiment, the treatment comprises silencing TGFBi in astrocytes by using a siRNA and/or sequestering TGFBi (for instance produced by altered astrocytes as a results of trauma) by using a neutralizing antibody against TGFBi. In the same way, the present invention also refers to a method for treating NHO which comprises administering to the patient a therapeutically effective dose or amount of a TGFBi inhibitor. In addition, siRNA inhibition of TGFBi could also be used to ensure that tissues affected by astrocyte- or nerve-conditioned media do not produce TGFBi either. So, it is possible to eliminate the TGFBi produced by astrocytes in trauma patients, but it is equally possible to prevent them from producing it using siRNA. But, in addition to that, the TGFBi produced once it reaches a target tissue is capable of self-induction, so the use of both the antibody and the siRNA could stop the self-amplification of its expression in tissues away from the brain or CNS.The objective of silencing TGFBi in astrocytes is avoiding the production of this protein. The results provided herein postulate that astrocytes are responsible for the release of molecules (including TGFBi) that enhance bone metabolism to the detriment of adipogenic metabolism. Therefore, it is herein suggested that astrocytes may be partly responsible for and involved in the development of NHO. This would be applicable to peripheral nerves, since, as it is herein shown, they secrete TGFBi and increase the expression of this protein in osteoblastic differentiation. By silencing TGFBi in astrocytes, astrocytes would no longer produce TGFBi and, therefore, one would expect that the effects of ACM on osteoblastic differentiation would be reduced. On the other hand, an alternative therapeutic strategy would be sequestering TGFBi (for instance TGFBi produced by altered astrocytes as a results of trauma which is present in the secretome) with the use of a neutralizing antibody against TGFBi. Thus, if the antibody sequesters TGFBi, it is expected that the effects of the ACM would not be as potent on osteoblast differentiation due to the lack of TGFBi.

It is well-known that if TBI occurs at the same time of other distal lesions, like fracture of a long bone, the risk of heterotopic ossification is highly increased. Normally, when a TBI occurs, the blood-brain barrier is compromised, and factors secreted by astrocytes (like TGBI) reach peripheral structures. In this situation the secretome of these astrocytes, would synergize with the osteoinductive factors (like TGFBI) released by peripheral structures (like the peripheral nerves) to promote abnormal bone anabolism

Thus, in a preferred aspect, the administration of the therapy (for instance siRNAs or naturalizing antibodies) can be carried out systemically, e.g., intravenously, to reduce the amounts of TGFBi systemically reducing the synergism between central and peripheral lesion on bone anabolism.

Alternatively, it would be possible to administer this treatment locally or intra-articularly in patients who have already developed the bone plaque. The use of this treatment after or during the surgery to excise the ectopic bone would reduce the potential recurrences of the ectopic bone formation.

Finally, the present invention also refers to an *in vitro* method for identifying compounds for the treatment of NHO, which comprises a) determining if the inhibition of TGFBi or the elimination of the anabolic effects of astrocytes on osteoblasts has taken place by the candidate compound, and b) wherein if said inhibition of TGFBi or the elimination of the anabolic effects of astrocytes on osteoblasts has taken place, it is indicative of the candidate may be effective in the treatment of NHO.

In a preferred embodiment, the elimination of the anabolic effects of astrocytes on osteoblasts is determined by confirming the absence of bone related markers such as: SPP1 and BMP2.

On the other hand, the present invention also refers to:
A method for detecting TGFBi in a test sample from a human subject at risk of developing NHO, the method comprising: (a) contacting the test sample with a reagent specific to TGFBi, (b) amplifying the TGFBi biomarker to produce an amplification product in the test sample; and (c) measuring TGFBi expression level by determining the level of the amplification product in the test sample.

In a preferred embodiment, the present invention is a computer-implemented invention, wherein a processing unit (hardware) and a software are configured to:
- Receive the expression level values of TGFBi,
- Process the expression level values of TGFBi for finding substantial variations or deviations, and
- Provide an output through a terminal display of the variation or deviation of the expression level, wherein the variation or deviation of the expression level indicates that the subject may be suffering from NHO.

For the purpose of the present invention the following terms are defined:
- According to the present invention, a reference value can be a "pre-established threshold value" or a "cut-off' value. Typically, a "threshold value" or "cut-off value" can be determined experimentally, empirically, or theoretically. According to the present invention, the "pre-established threshold" value refers to a value previously determined in subjects who are nonsuffering from NHO or healthy subjects. Thus, for instance, the subject is likely to suffer from NHO, or to have a poor prognosis, when a higher expression of TGFBi is identified, as compared with a pre-established "threshold value". A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of". Thus, the phrase "consisting of" indicates that the listed elements are required or mandatory, and that no other elements may be present.
- By "therapeutically effective dose or amount" of a TGFBi inhibitor is intended an amount that, when administered as described herein, brings about a positive therapeutic response in a subject having NHO. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

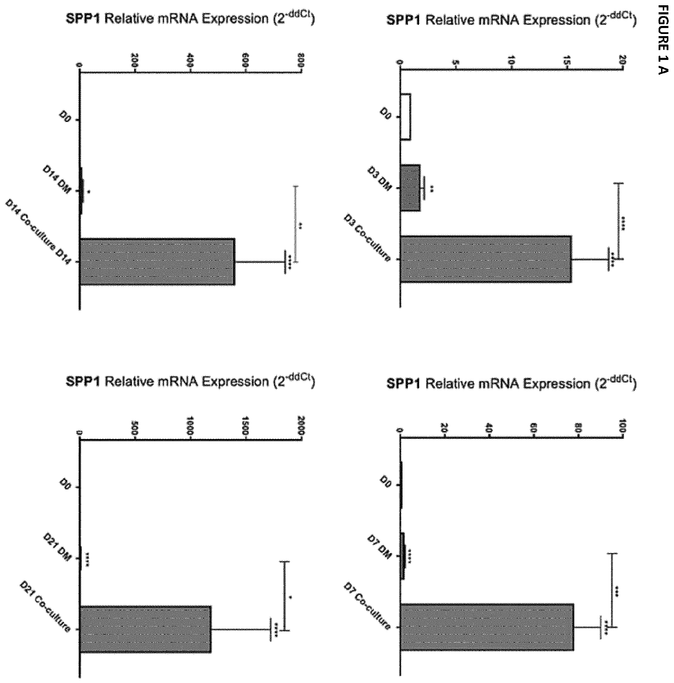

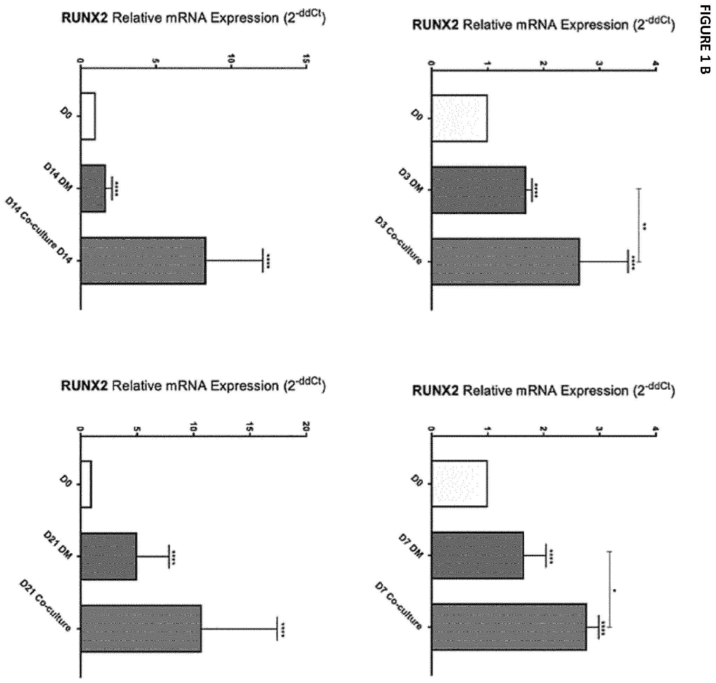

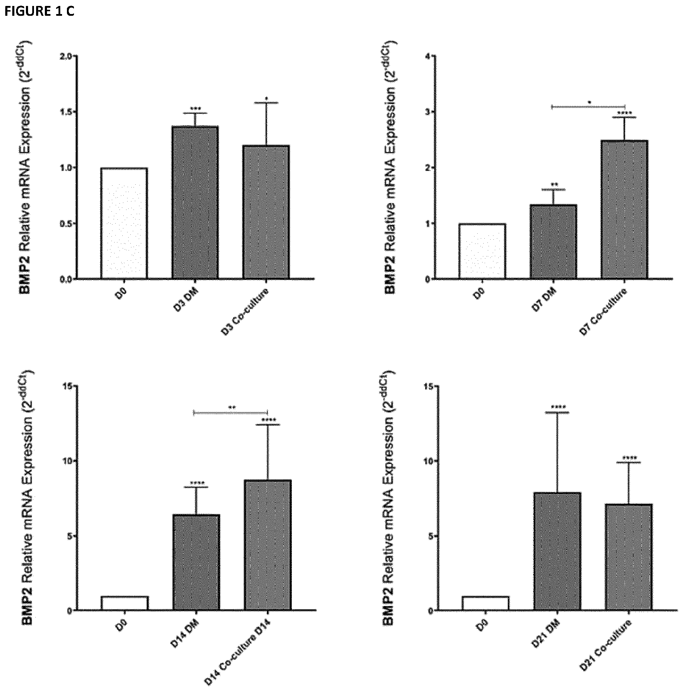

**Figure 1****. Impact of astrocyte-osteoblast coculture on osteoblastogenesis.** A-C) Expression of the osteoblastogenic differentiation markers SPP1, RUNX2, and BMP2 measured by RT-PCR after twenty-one days of cell coculture of astrocytes and osteoblasts.

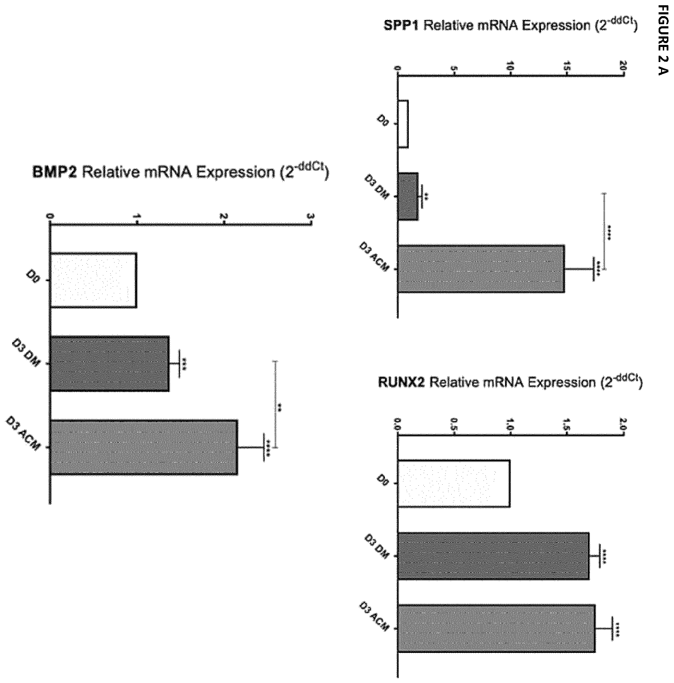

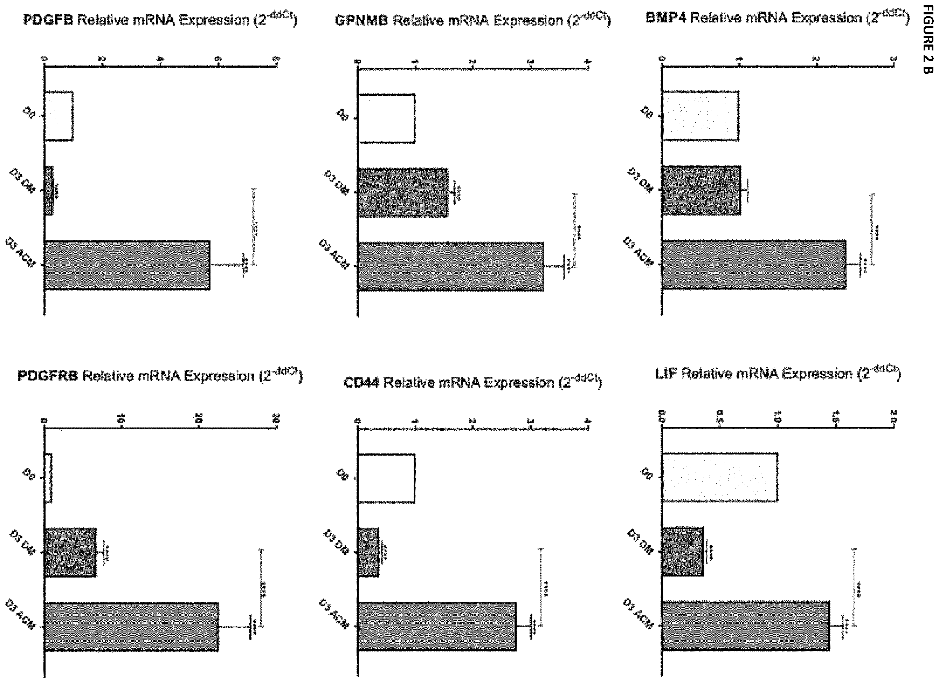

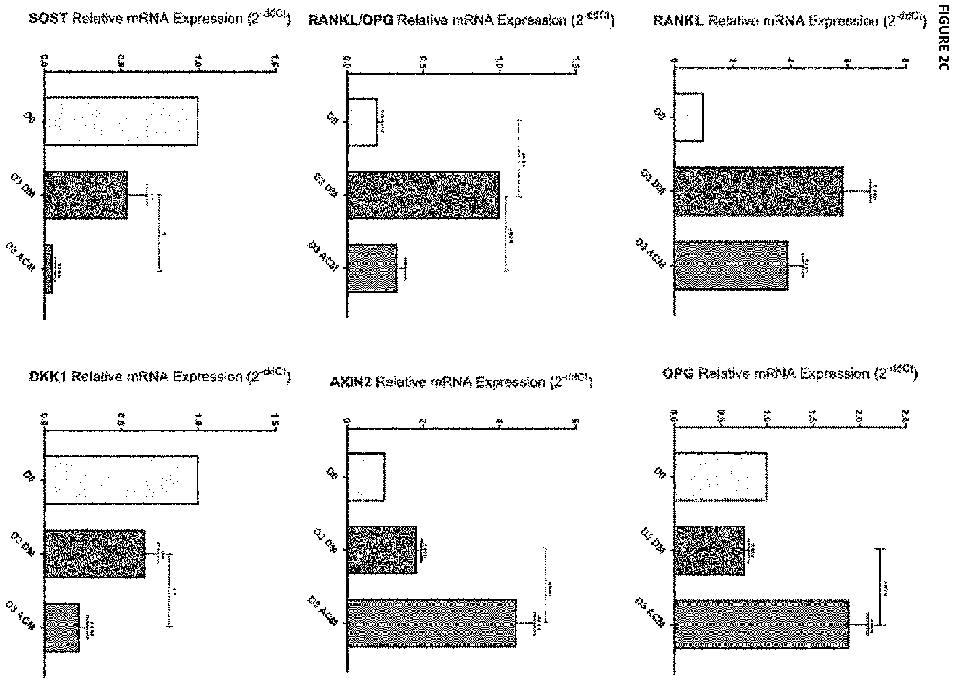

**Figure 2****. Effect of MCA on bone anabolism in the osteoblastogenesis process.** A) Expression of the osteoblastogenic differentiation markers SPP1, RUNX2 and BMP2 measured by RT-PCR in ACM-stimulated osteoblasts differentiated into osteoblasts for three days. B-C) Quantification by RT-PCR of the expression of markers related to bone metabolism BMP4, LIF, GPNMB, CD44, PDGFβ, PDGFRβ, RANKL, OPG, RANKL/OPG ratio, and the WNT-related genes AXIN2, SOST, and DKK1 during SaOS2 differentiation into osteoblasts in the presence or absence of MCA for three days.

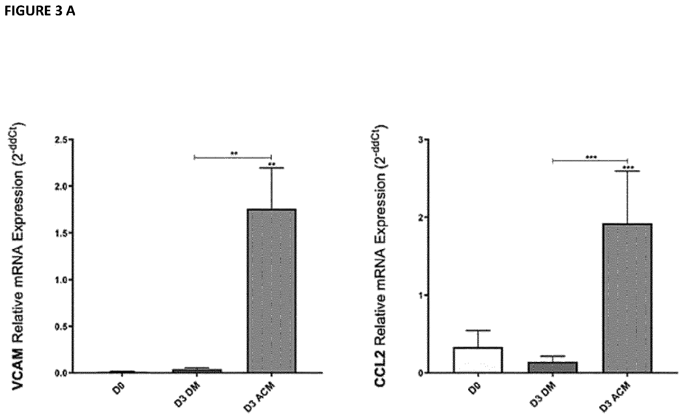

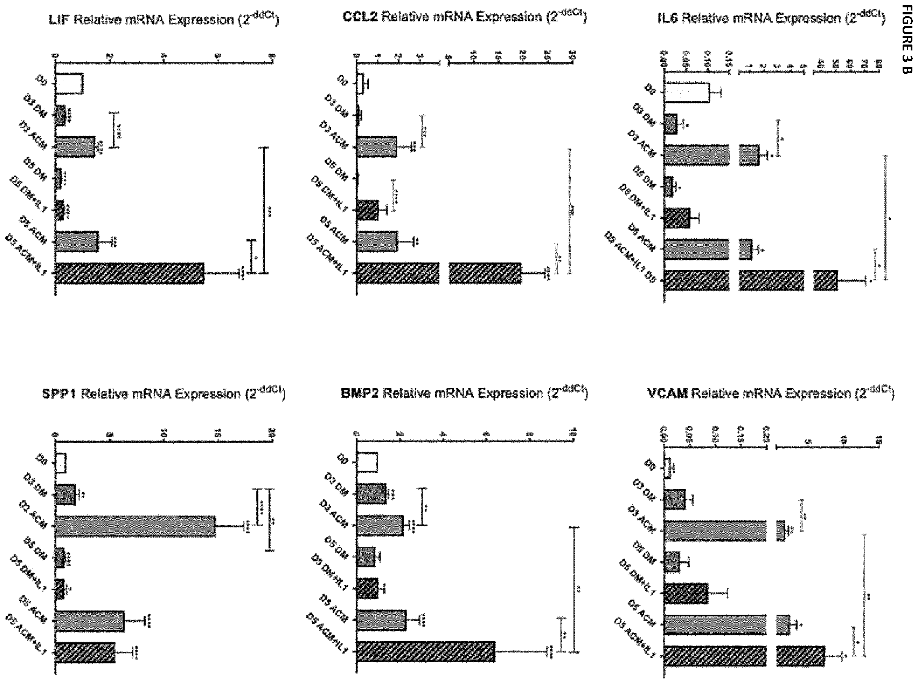

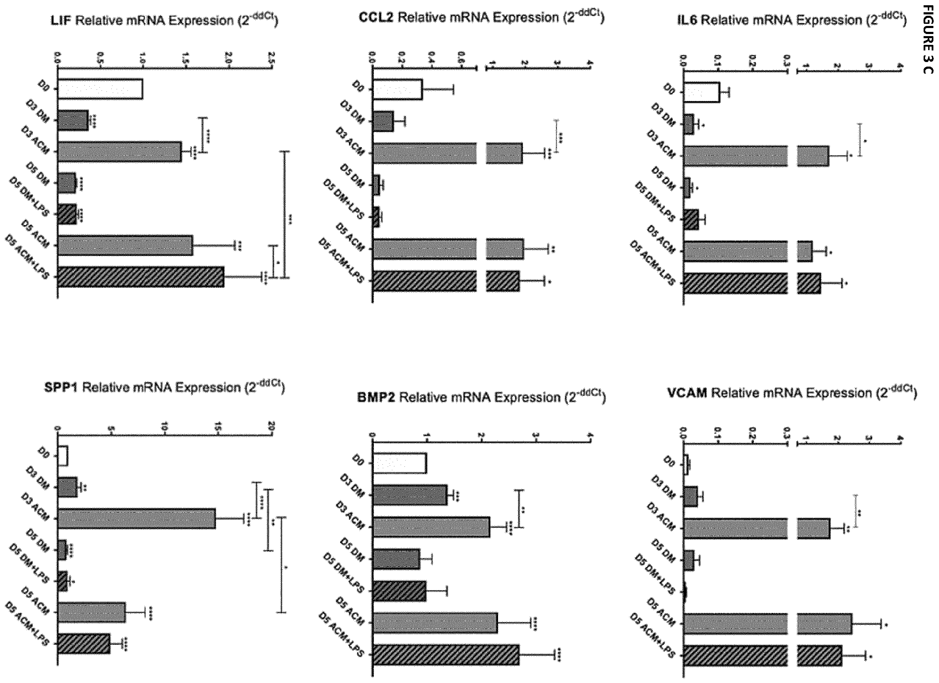

**Figure 3****. Effect of MCA o inflammatory responses.** A) Expression of the inflammatory markers VCAM and CCL2 measured by RT-PCR in SaOS2 differentiated into osteoblasts for three days in the presence and absence of MCA. B-C) Effect of the addition of IL1β or LPS on the basal effects of MCA on osteoblastogenesis. The inflammatory and bone metabolism markers IL6, VCAM, CCL2, BMP2, LIF, and SPP1 were measured by RT-PCR of in SaOS2 cells stimulated with IL1β (0.1 ng/mL) or LPS (100 ng/mL) for 48h, after being differentiated into osteoblasts in presence and absence of the MCA for three days.

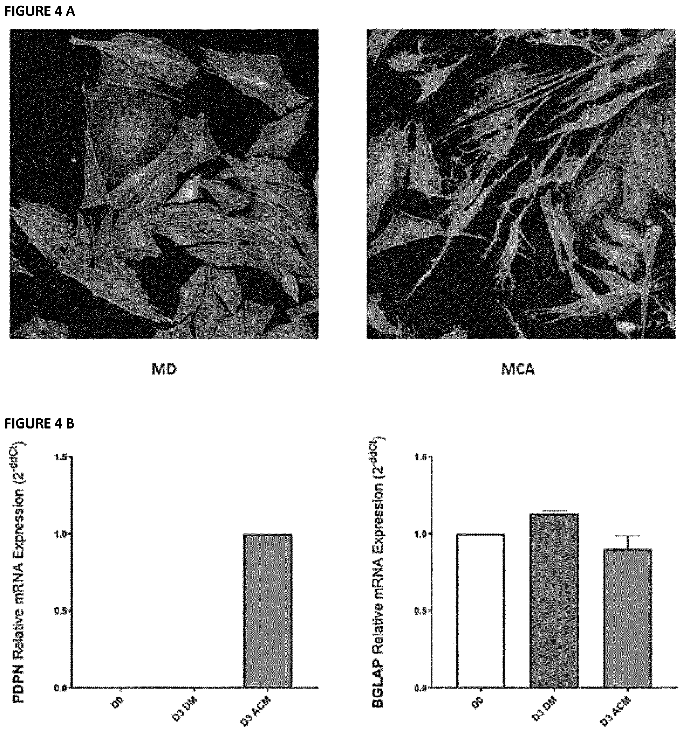

**Figure 4****. Morphological changes on osteoblasts induced by MCA.** A) Tubulin, actin and cell nucleus staining of SaOS2 cells differentiated for three days in the presence and absence of ACM. B) Gene expression determined by RT-PCR of the osteocyte markers PDPN and BGLAP in SaOS2 cells differentiated into osteoblasts for three days in the presence or absence of ACM.

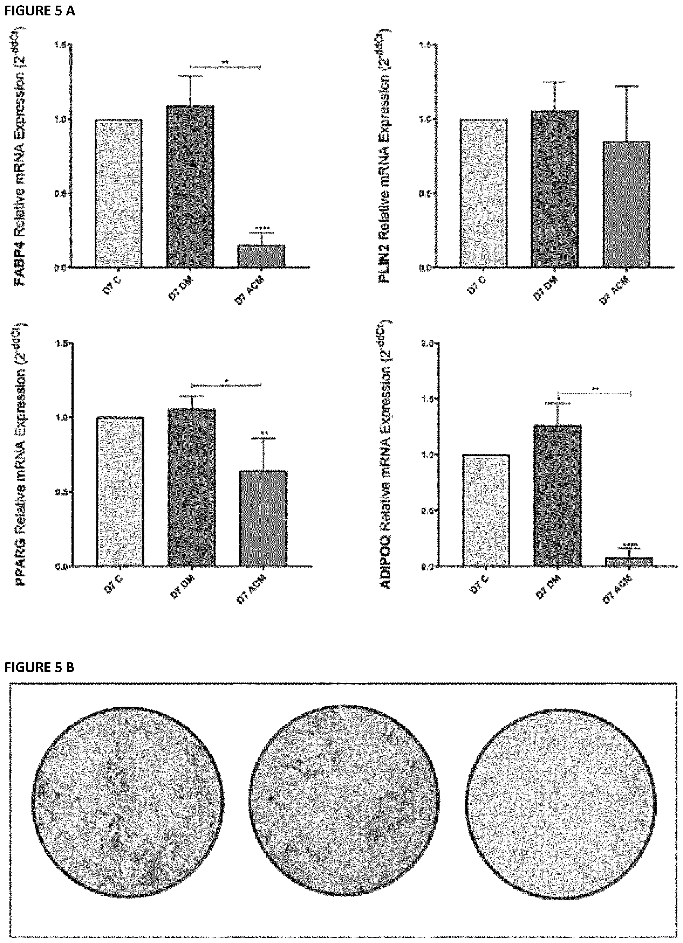

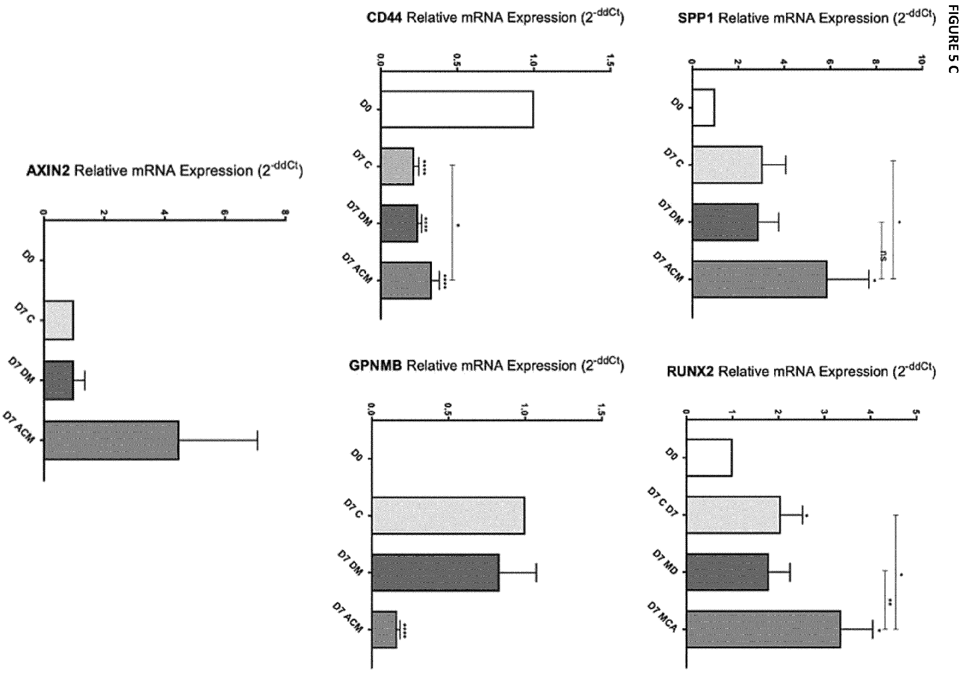

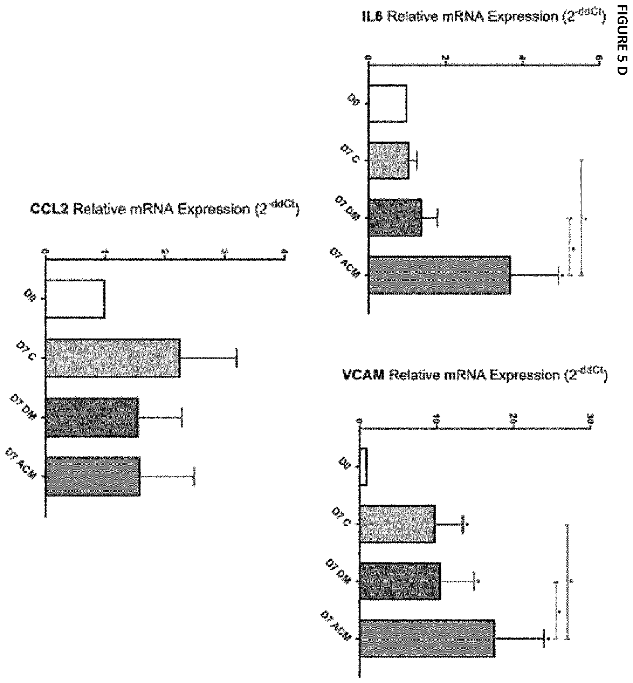

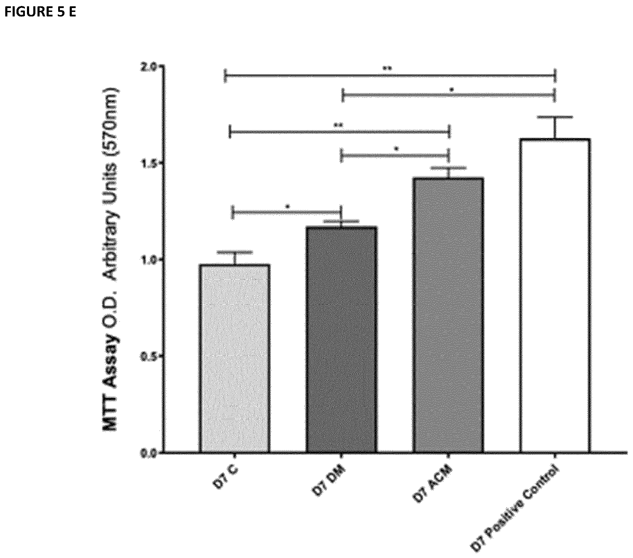

**Figure 5****. Effect of ACM on adipogenesis.** A) Study of adipogenic markers. Gene expression measured by RT-PCR of the main adipogenic markers FABP4, PLIN2, PPARG and ADIPOQ in C3H10T1/T2 cells differentiated into adipocytes for seven days in the presence or absence of ACM. B) Oil Red staining of the lipid droplets during the C3H10T1/2 differentiation into adipocytes for 7 days in the presence or absence of DM and ACM. C) Study of anabolic markers. Gene expression measured by RT-PCR of the bone-related markers SPP1, RUNX2, CD44, GPNMB, and AXIN2 in C3H10T1/2 differentiated for seven days in the presence or absence of ACM. D) Study of inflammatory markers. Determination by RT-PCR of the inflammatory markers IL6, VCAM, and CCL2 during the adipogenesis process in the presence or absence of the ACM. E) Cell viability study using the MTT assay of C3H10T1/2 differentiated into adipocytes for seven days in the presence or absence of ACM.

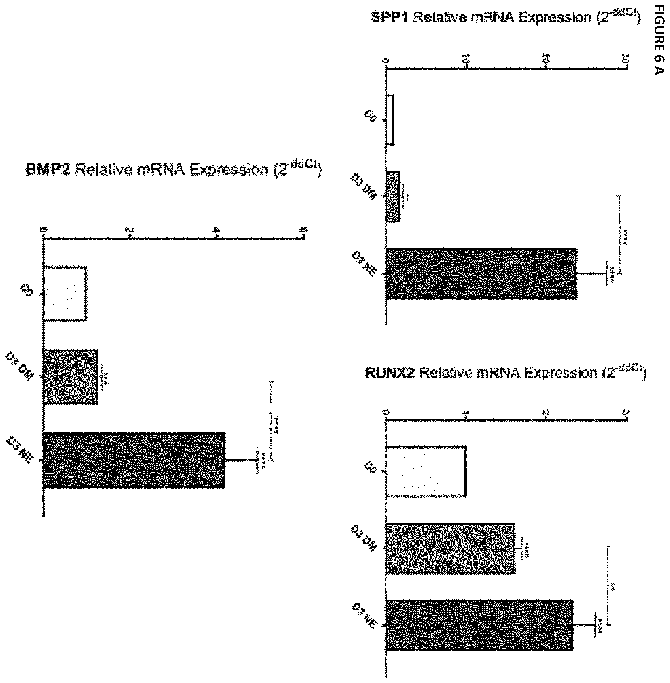

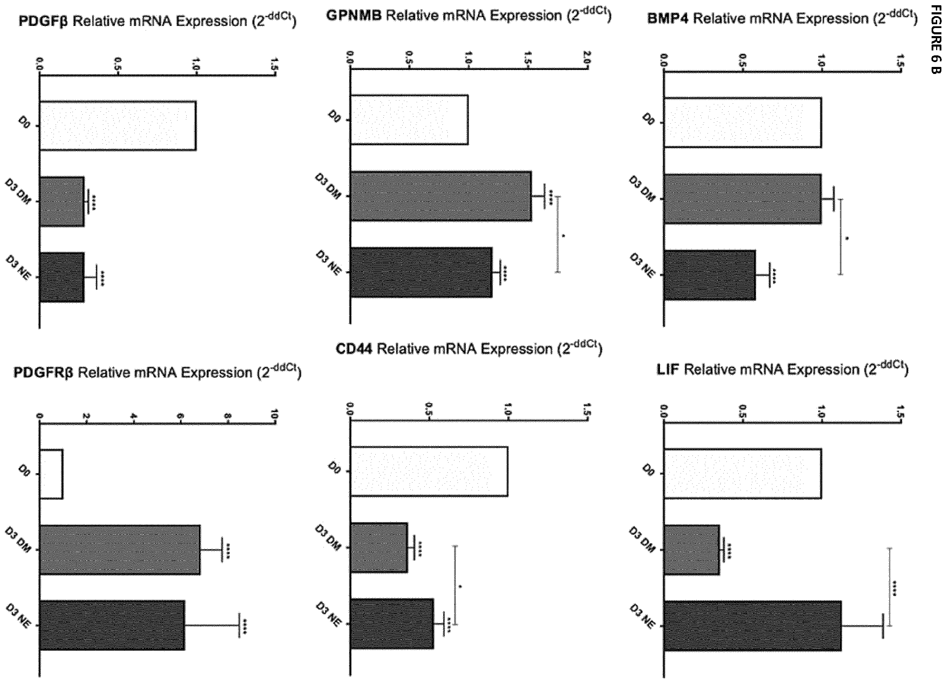

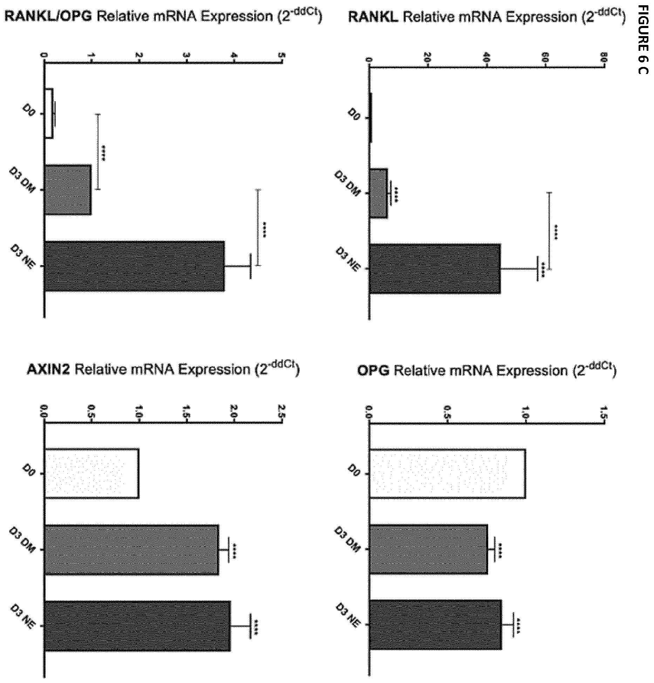

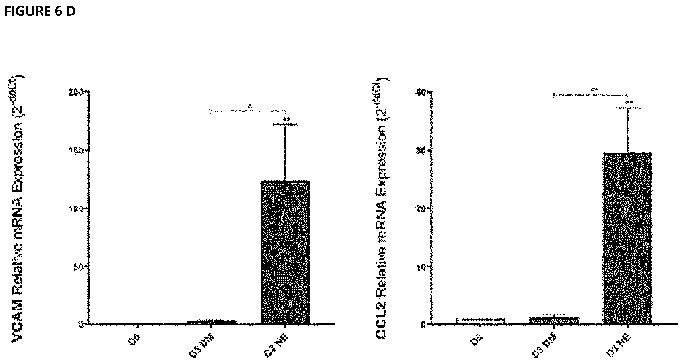

**Figure 6****. Effect of NE on osteoblastogenesis.** A) Gene expression measured by RT-PCR of the bone-related markers SPP1, RUNX2 and BMP2 after three days of the NE-SaOS2 coculture during osteoblast differentiation. B-C) Determination by RT-PCR of the expression of bone-related markers BMP4, LIF, GPNMB, CD44, PDGFβ, PDGFRβ, RANKL, OPG, RANKL/OPG ratio, and AXIN2 during SaOS2 differentiation into osteoblasts for three days in the presence or absence of the NE. D) Gene expression measured by RT-PCR of the inflammatory markers VCAM and CCL2 during osteoblastic differentiation of SaOS2 in the presence or absence of the NE for three days.

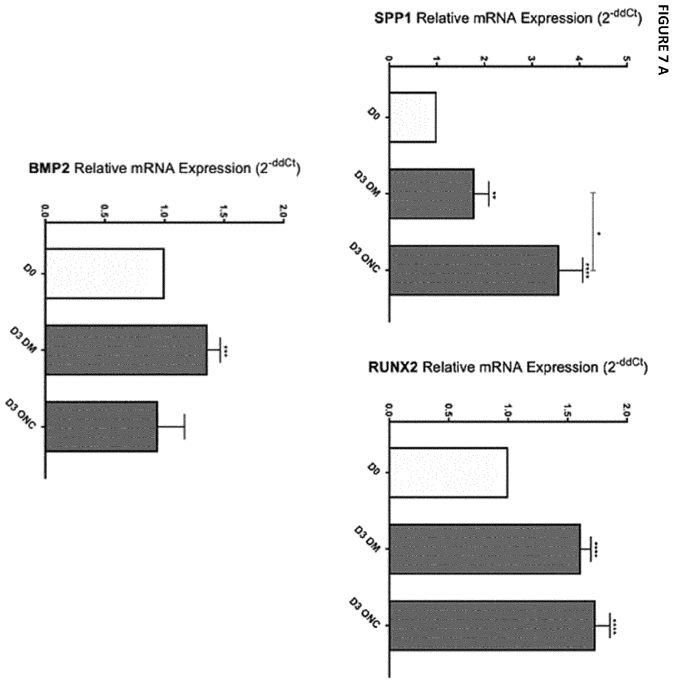

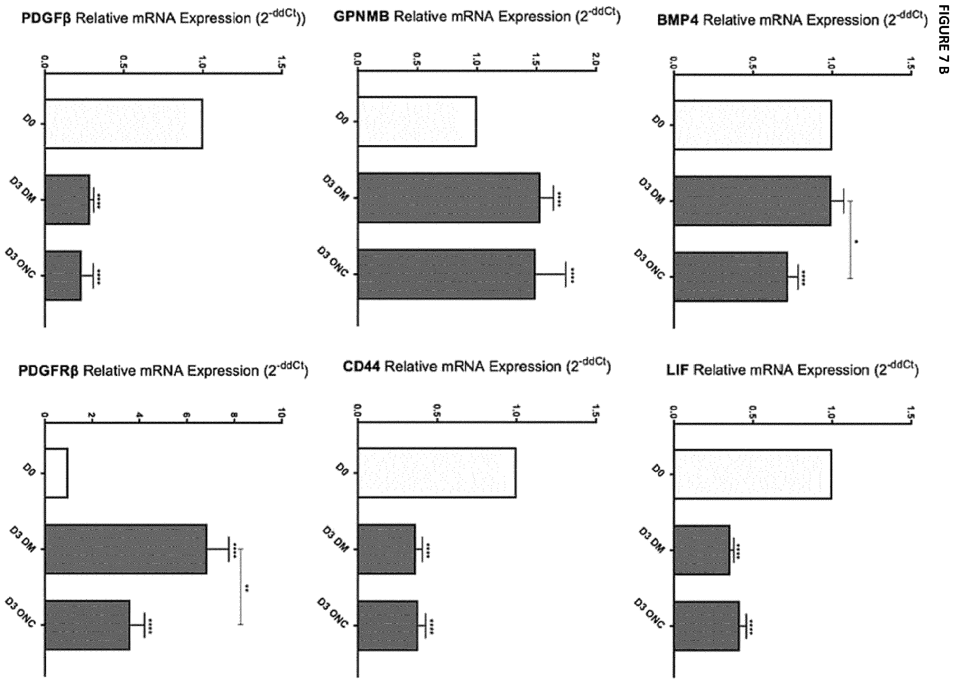

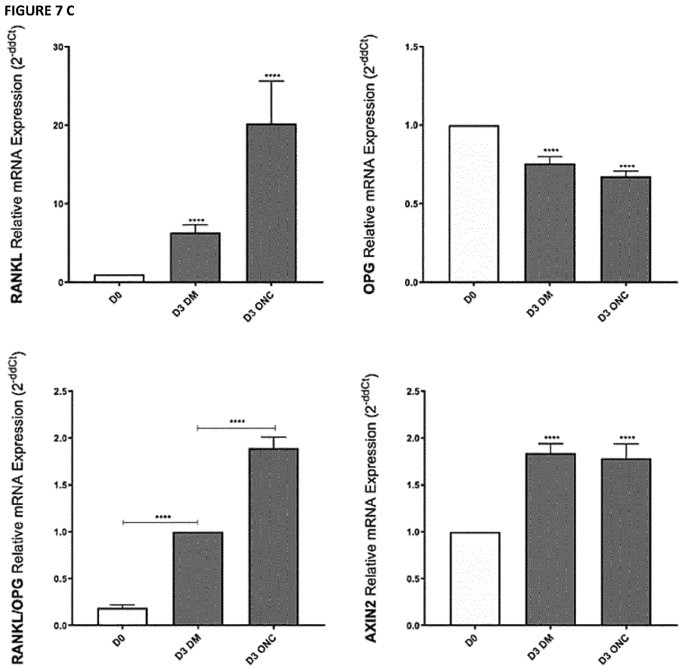

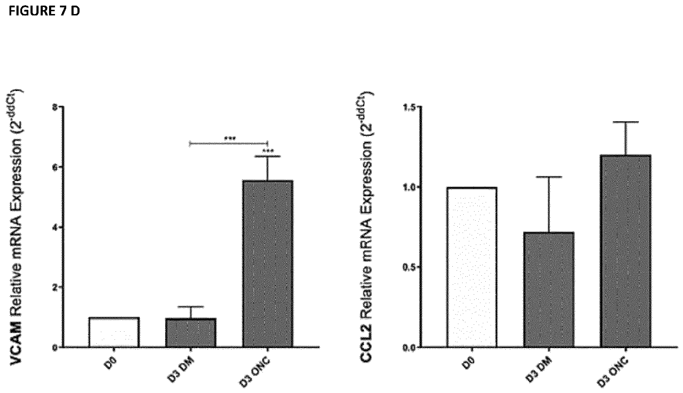

**Figure 7****. Impact of ONC on osteoblastogenesis.** A) Quantification by RT-PCR of the gene expression of SPP1, RUNX2 and BMP2 in SaOS2 differentiated into osteoblasts for three days in the presence or absence of the ONC. B-D) Determination of gene expression by RT-PCR of bone-related and inflammatory markers BMP4, LIF, GPNMB, CD44, PDGFβ, PDGFRβ, RANKL, OPG, RANKL/OPG ratio, AXIN2, VCAM and CCL2 in SaOS2 cells differentiated into osteoblasts for three days in the presence or absence of ONC.

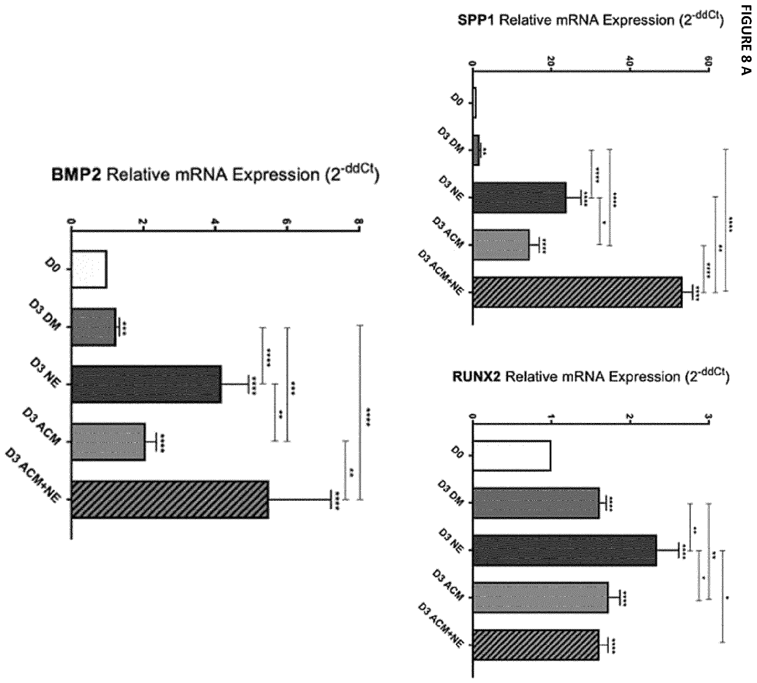

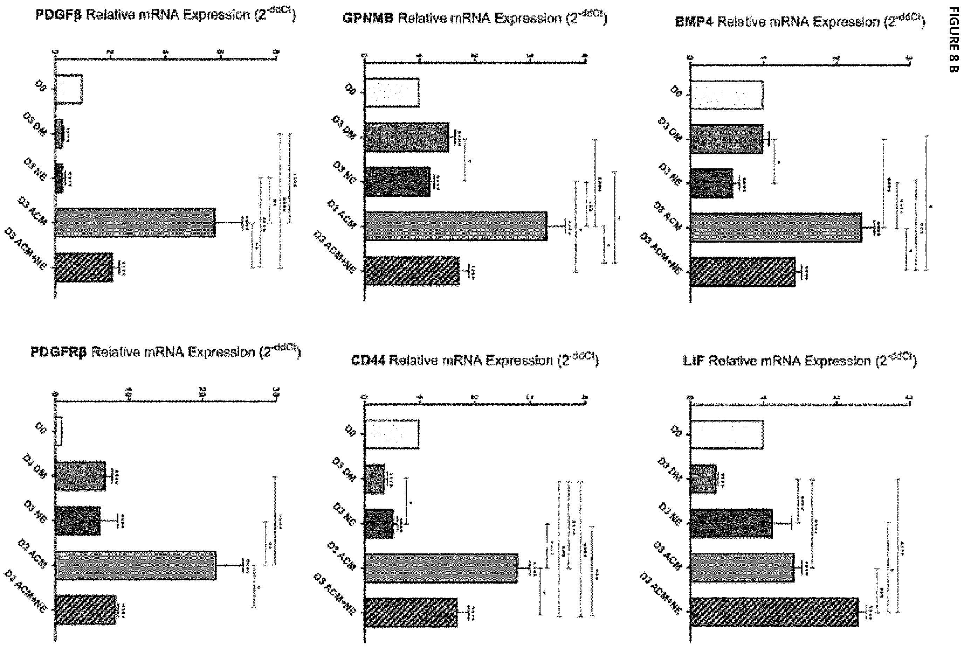

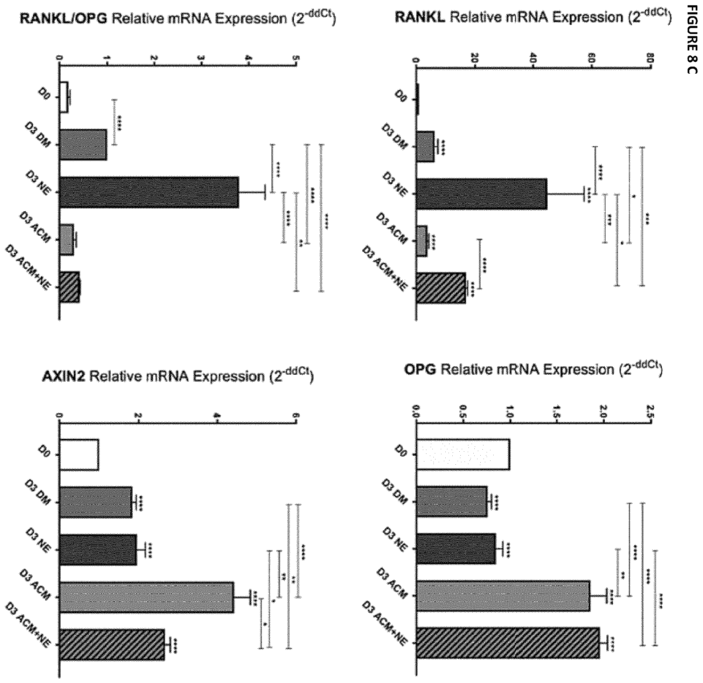

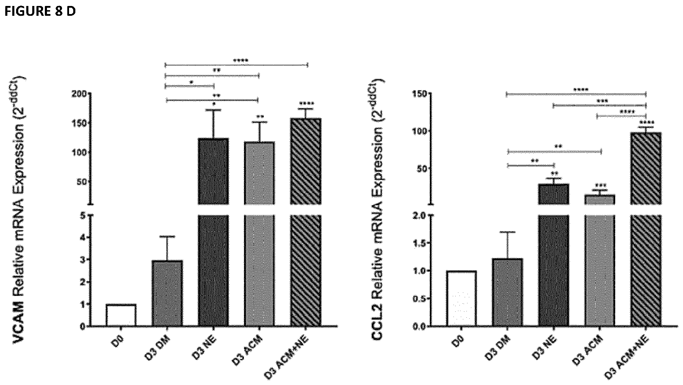

**Figure 8****. Effect of ACM on the NE-osteoblast co-culture.** A-D) Determination of gene expression by RT-PCR of the bone markers SPP1, RUNX2, BMP2, BMP4, LIF, GPNMB, CD44, PDGFβ, PDGFRβ, RANKL, OPG, RANKL/OPG ratio, AXIN2, VCAM and CCL2 in SaOS2 cocultured with the NE for three days in the presence or absence of ACM.

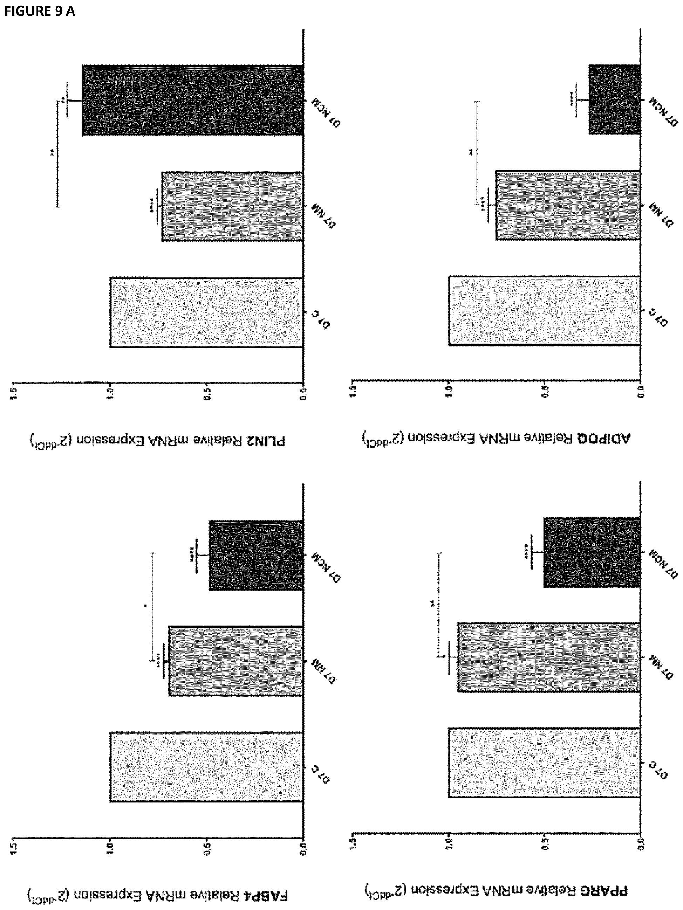

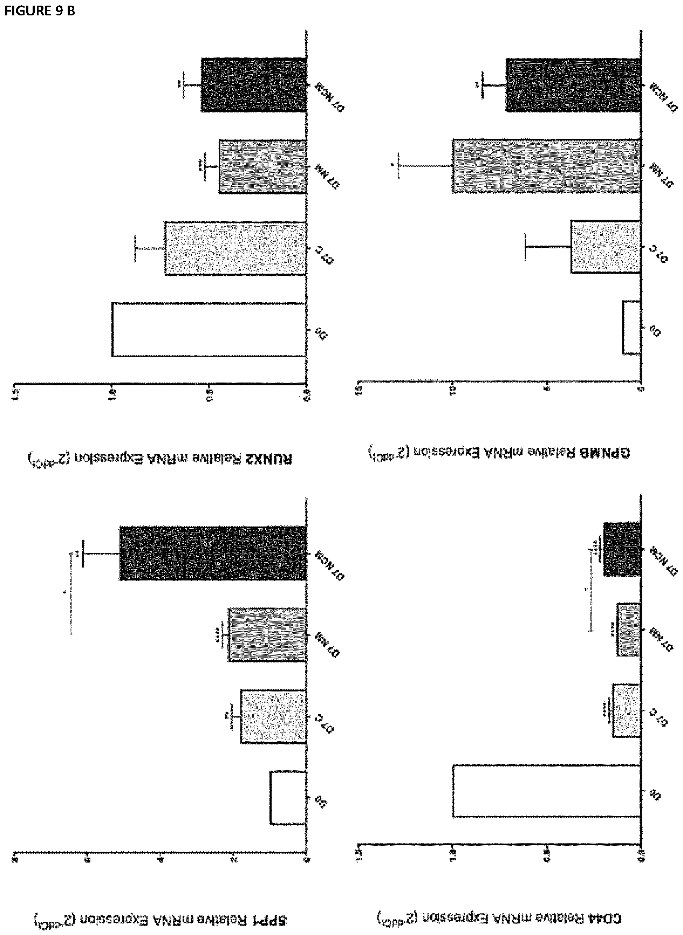

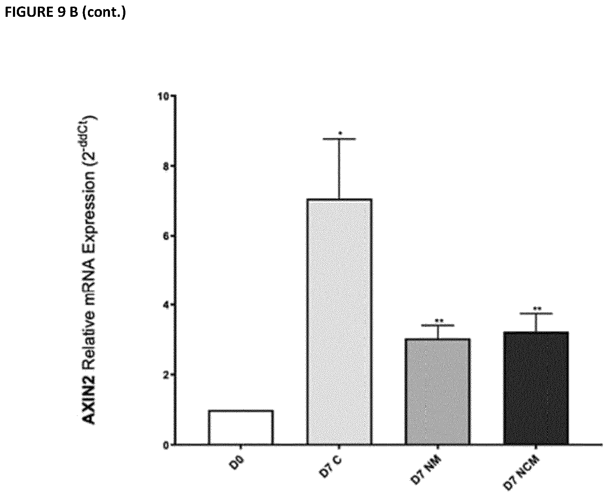

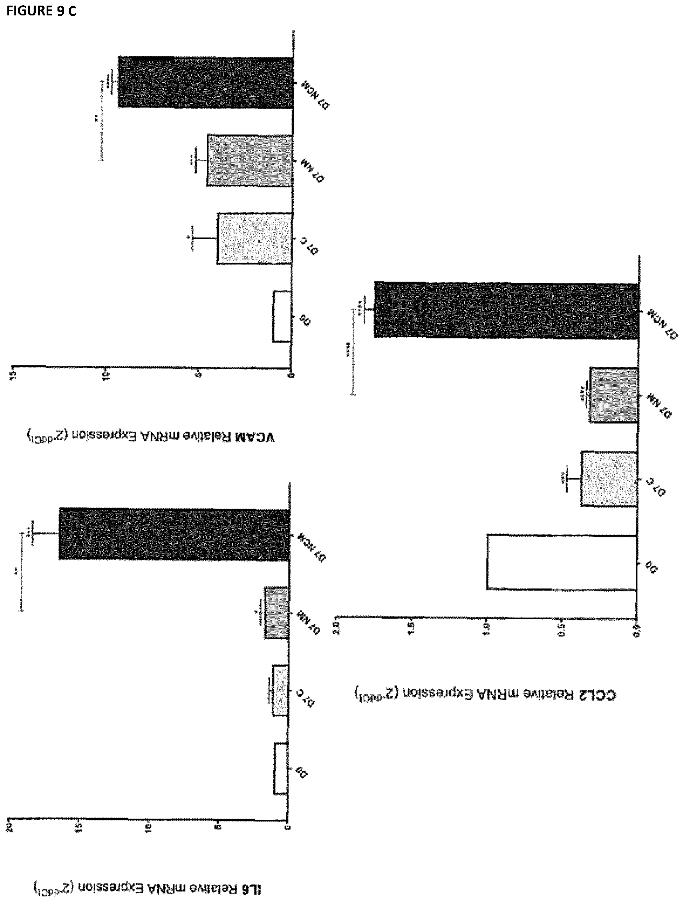

**Figure 9****. Effect of NCM on adipogenesis.** A) Expression of the main adipogenic markers FABP4, PLIN2, PPARG, and ADIPOQ quantified by RT-PCR in C3H10T1/2 differentiation into adipocytes for seven days in the presence and absence of NCM. B-C) Quantification by RT-PCR of the gene expression of SPP1, RUNX2, CD44, GPNMB, AXIN2, IL6, VCAM, and CCL2 in C3H10T1/2 cells differentiated into adipocytes for seven days in the presence or absence of NCM.

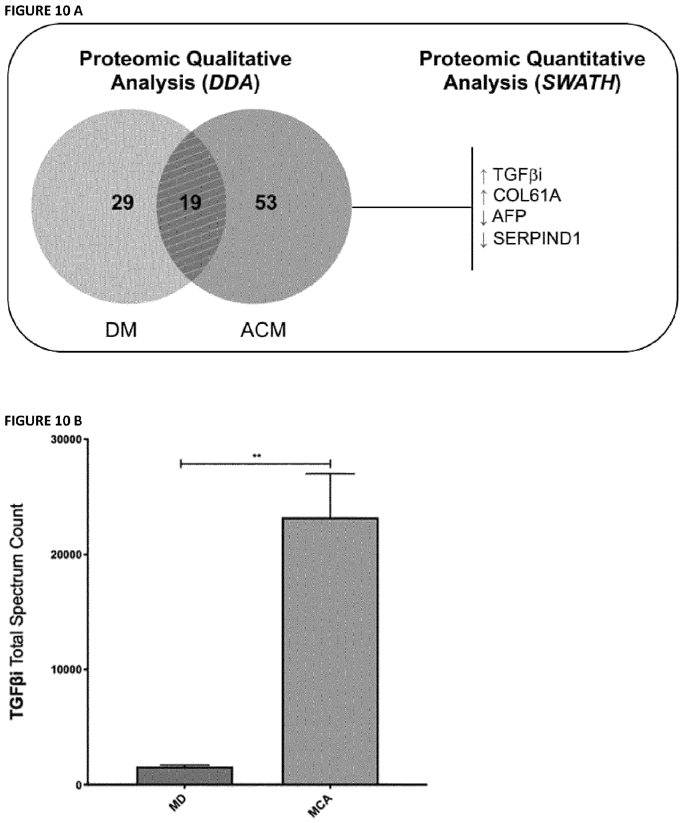

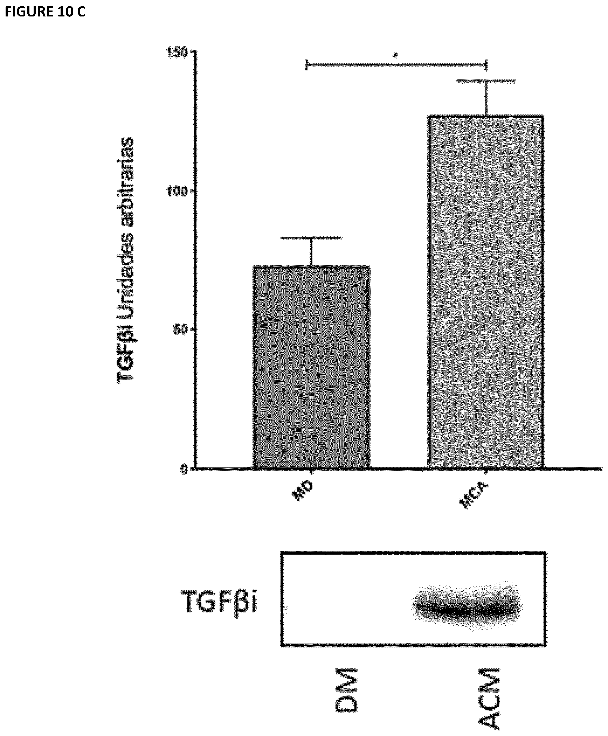

**Figure 10****. Characterization of the secretome of ACM and DM.** A) Qualitative (DDA) and quantitative (SWATH) analysis of the statistically significant proteins detected by proteomics studies in DM and ACM. B) Comparison of the total spectrum count obtained by quantitative proteomics studies (SWATH) of the expression of TGFβi in the DM and ACM. C) Protein expression of TGFβi of the DM and ACM measured by western blot.

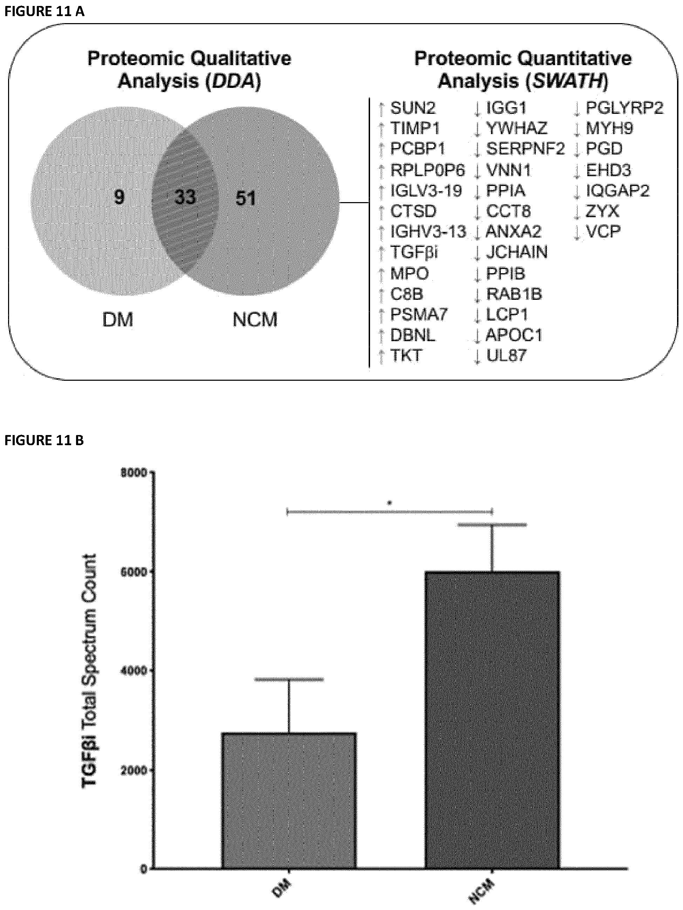

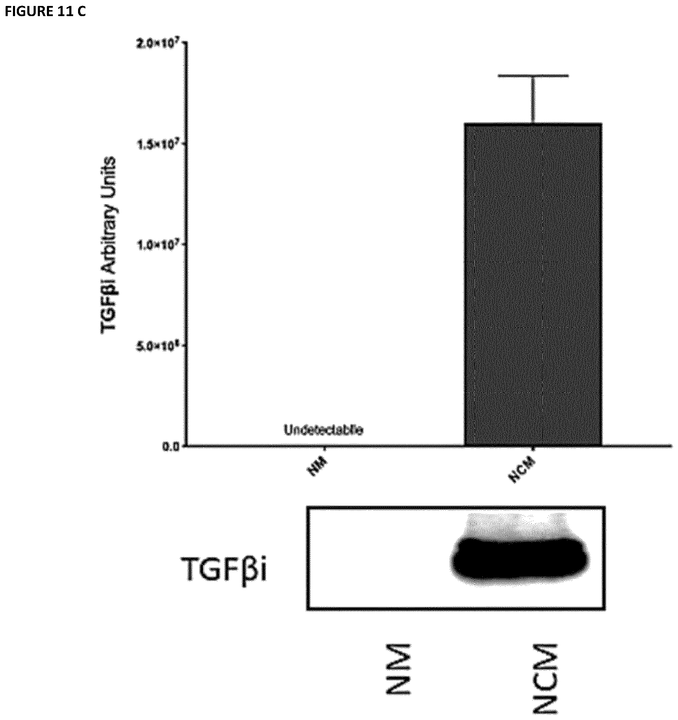

**Figure 11****. Analysis of the secretome and protein expression of the NCM and NM.** A) Qualitative (DDA) and quantitative (SWATH) analysis of statistically significant proteins detected by proteomics studies in the DM and NCM. B) Comparison of the results obtained by means of the quantitative proteomic study of the expression of TGFβi in the DM and the NCM. C) Protein expression of TGFβi in NM and ACM observed by western blot.

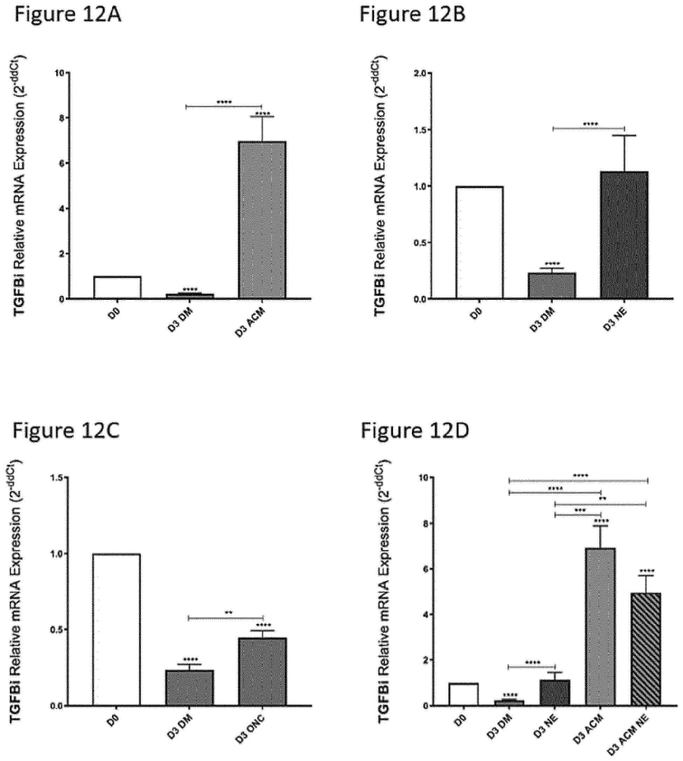

**Figure 12****. TGFβi gene expression in osteoblastogenesis.** A) TGFβi gene expression measured by RT-PCR in SaOS2 differentiation into osteoblast during three days in the presence or absence of ACM. B) TGFβi gene expression measured by RT-PCR of in SaOS2 differentiation into osteoblast for three days in the presence or absence of the NE. C) TGFβi gene expression quantified by RT-PCR in osteoblast differentiations in the presence or absence of ONC. D) TGFβi gene expression measured by RT-PCR in NE-SaOS2 differentiation into osteoblasts for three days in presence or absence of ACM.

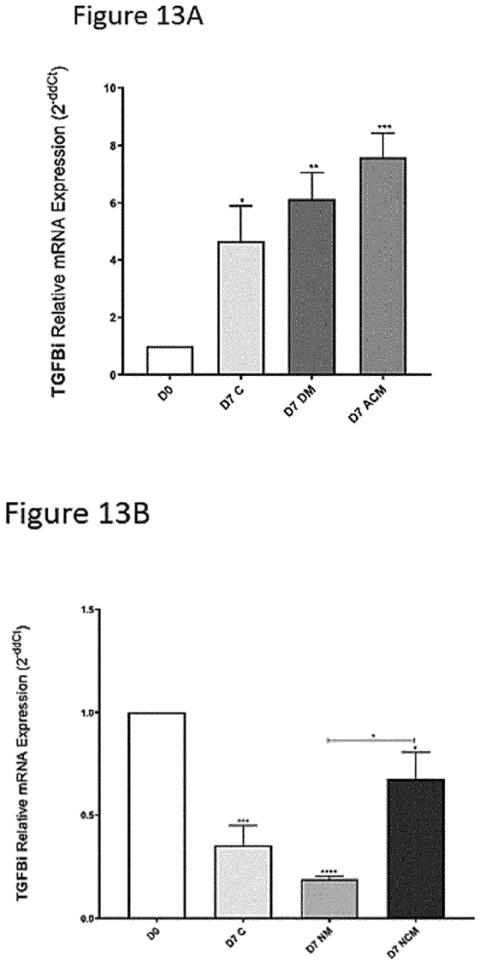

**Figure 13****. TGFβi** gene **expression in adipogenesis.** A) TGFβi gene expression measured by RT-PCR in C3H10T1/2 cells differentiated into adipocytes for seven days stimulated with ACM. B) TGFβi gene expression measured by RT-PCR in C3H10T1/2 differentiated into adipocytes for seven days in the presence or absence of NCM.

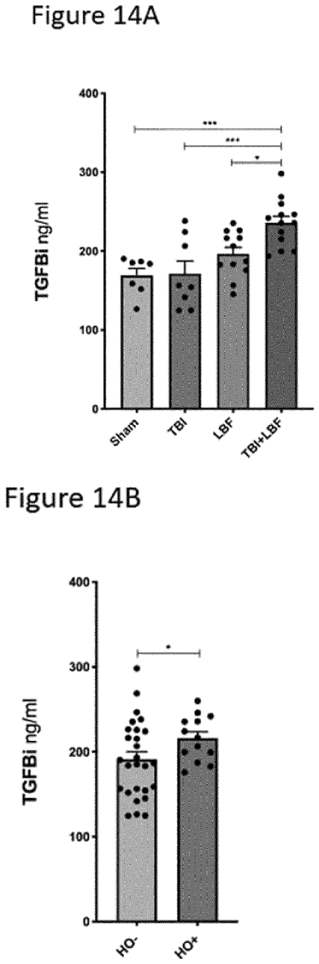

**Figure 14****. TGFβi protein expression in NHO *in vivo* model.** A) Plasma levels of TGFβi on TBI, LBF, TBI+LBF rats after two days of the injury. Results were compared versus controls. B) TGFβi plasma levels of on animals which developed the HO (including TBI+LBF and LBF) at 6 weeks post-injury.

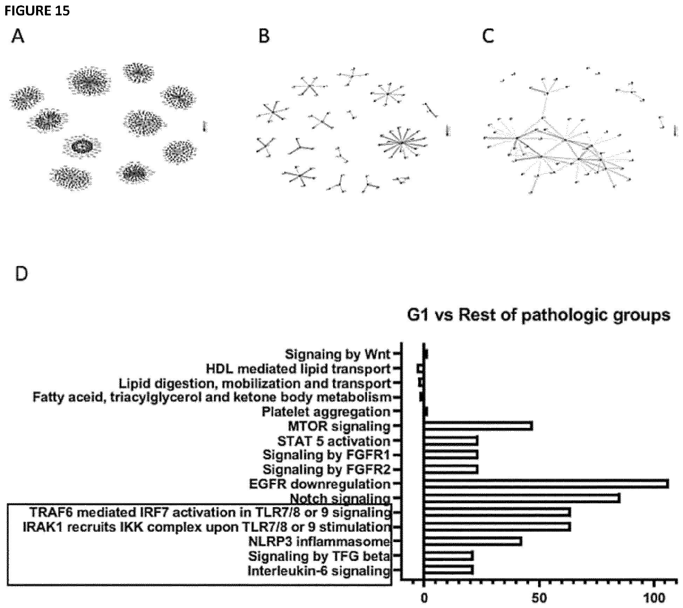

**Figure 15****. Correlations between the genes related to osteoblastogenesis and the proteomic analysis of the sera of the patients.** A-C) Correlations between gene expression measured by RT-PCR of SPP1, RUNX2, BMP2, BMP4, LIF, GPNMB, CD44, PDGFβ, PDGFRβ, RANKL, OPG, RANKL/OPG ratio, and AXIN2 in SaOS2 differentiation into osteoblasts in the presence or absence of the serum of the patients of G1 (A), G2 (B) and G3 (C) for three, seven, fourteen and twenty-one days, and the data obtained from the secretome of the respective patients by proteomics. D) Pathway-enrichment analysis of G1 patients secretome in relation to the other groups.

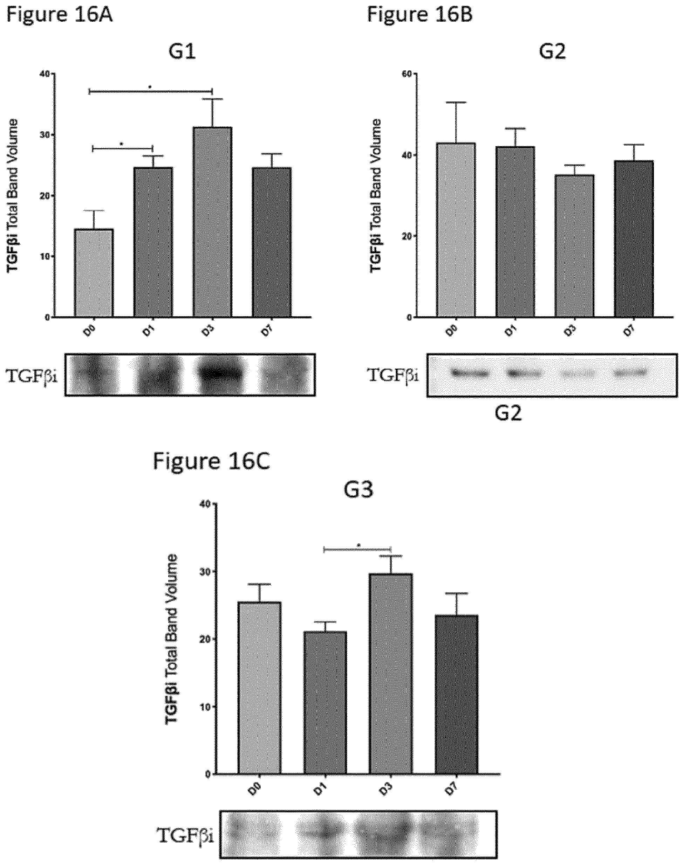

**Figure 16****. TGFβi protein expression in patients at risk of suffering from NHO.** A) Protein expression of TGFβi quantified by western blot of serum precipitates from patients who suffered TBI and FHL (G1). B) Determination of the expression of TGFβi by western blot in the precipitates from the serum of patients with TBI (G2). C) Quantification of serum TGFβi expression by western blot in patients who suffered FHL (G3).

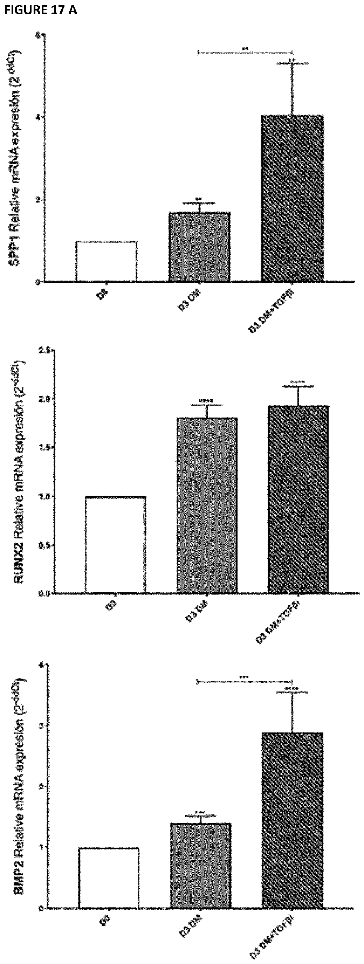

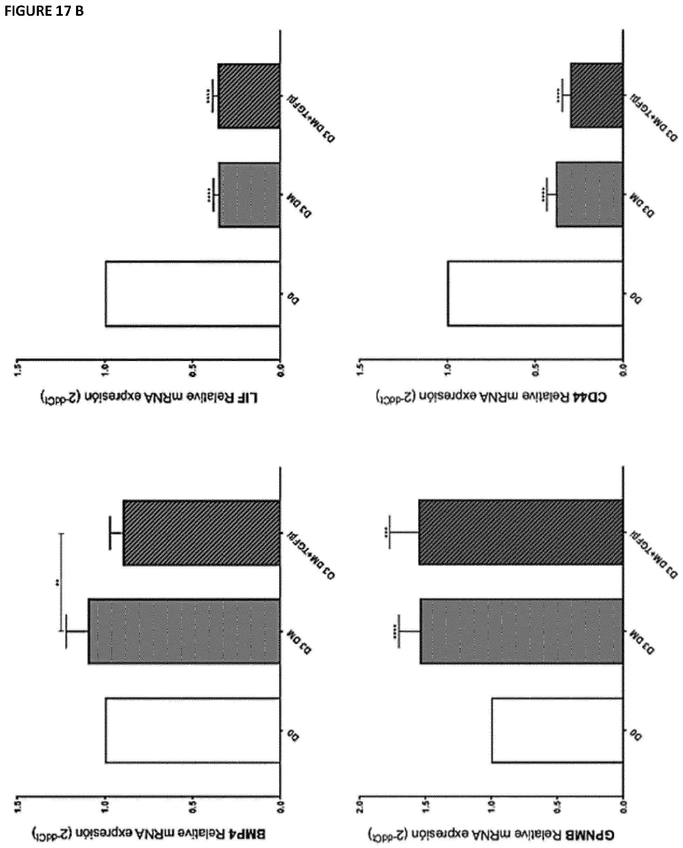

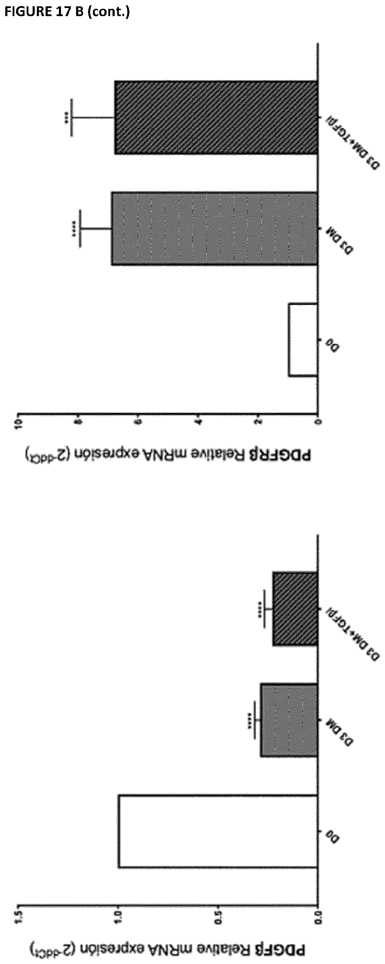

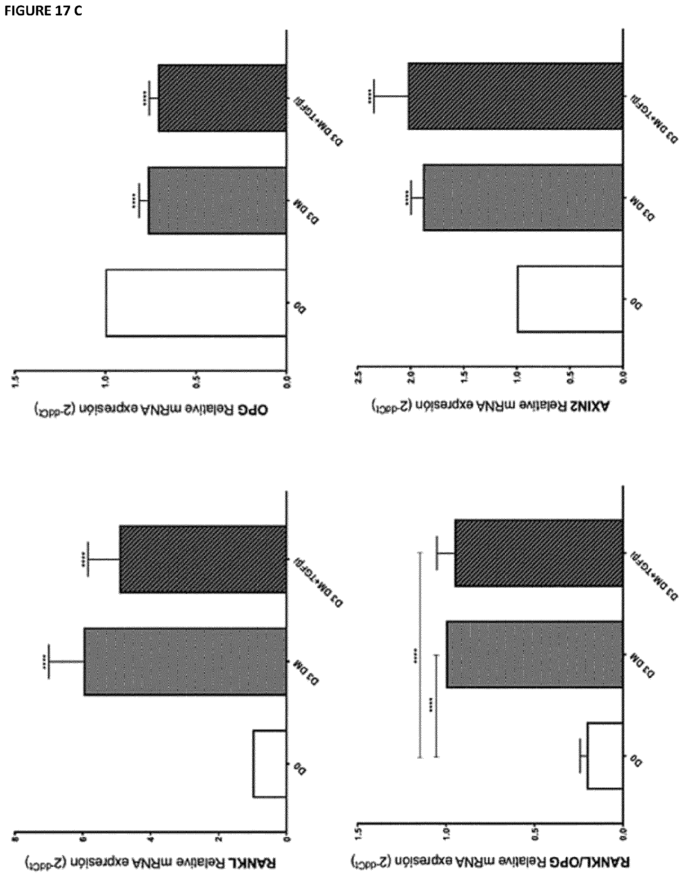

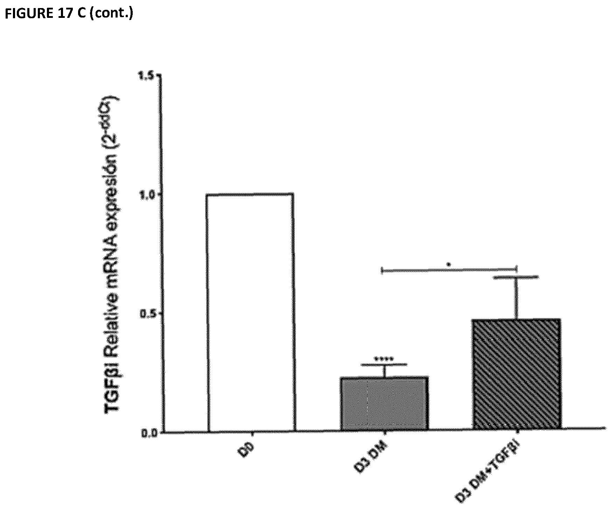

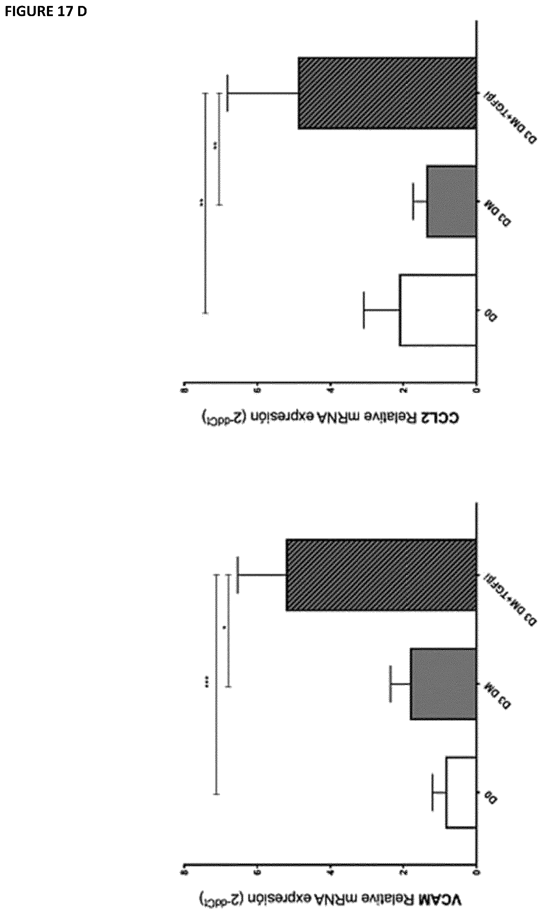

**Figure 17****. Effect of TGFβi stimulation on osteoblastogenesis.** A-D) Gene expression measured by RT-PCR of bone-related and inflammatory markers SPP1, RUNX2, BMP2, BMP4, LIF, GPNMB, CD44, PDGFβ, PDGFRβ, RANKL, OPG, RANKL/OPG ratio, AXIN2, TGFβi, VCAM and CCL2 in SaOS2 differentiated for three days to osteoblast in the presence or absence of TGFβi 3µg/mL.

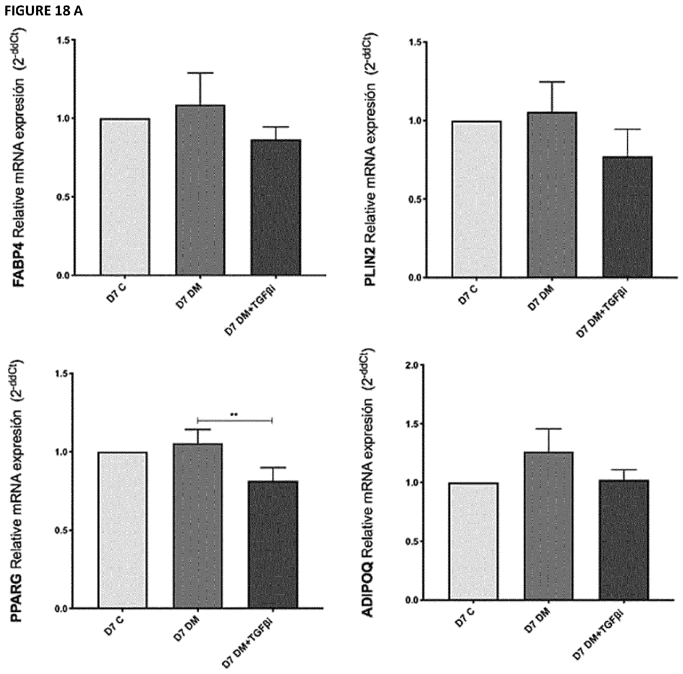

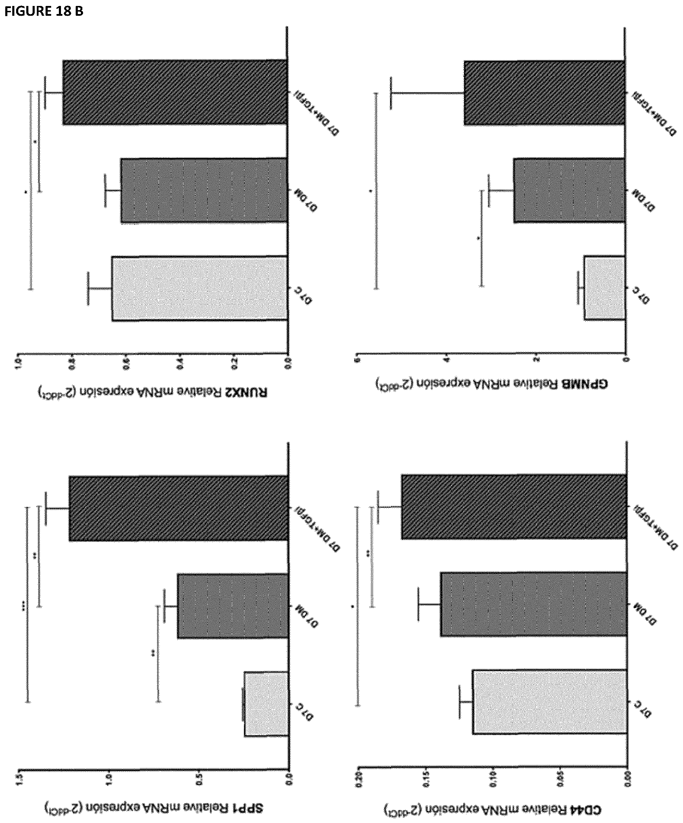

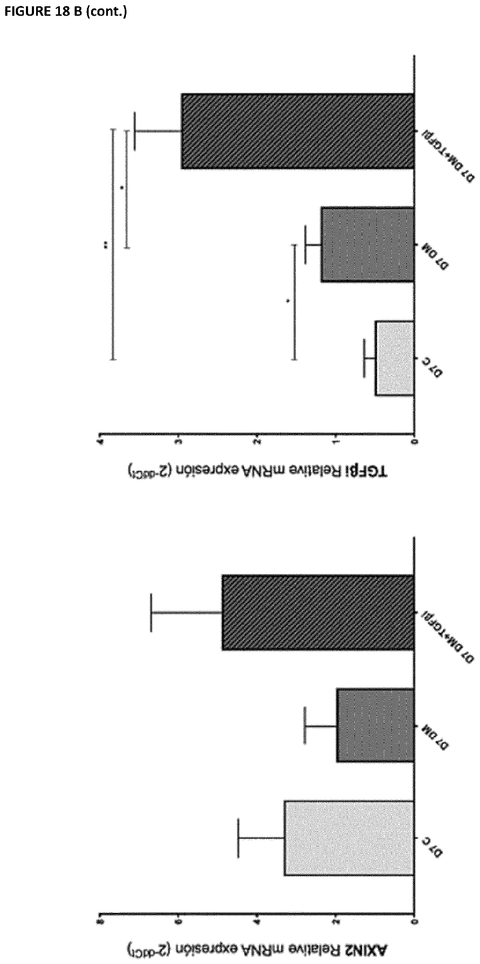

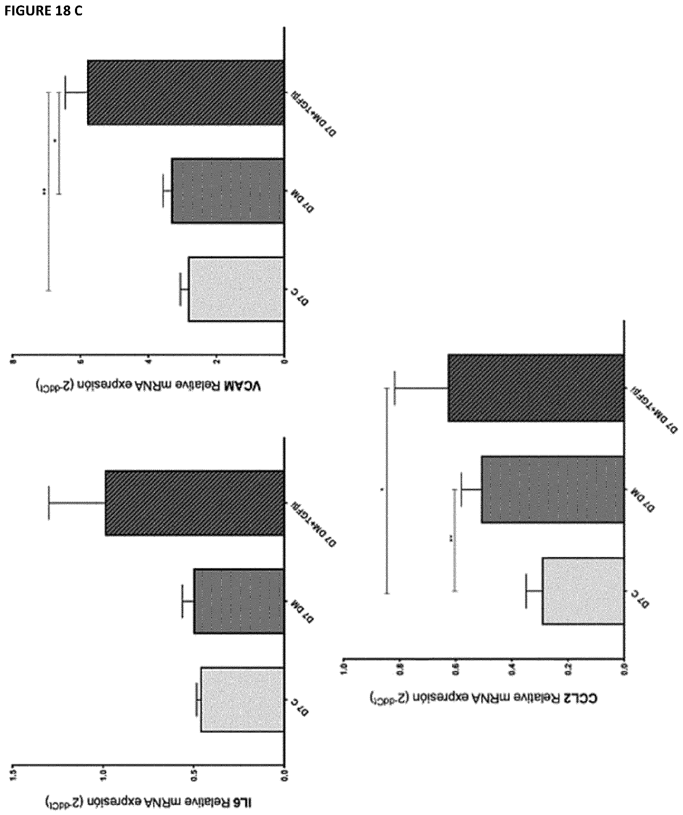

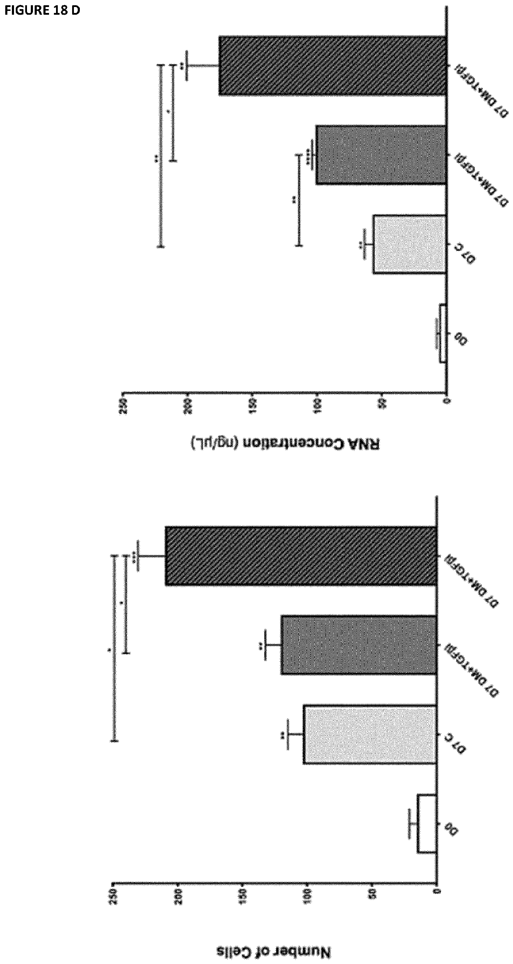

**Figure 18****. Effect of TGFβi stimulation on adipogenesis.** A) Gene expression measured by RT-PCR of the main adipogenic markers FABP4, PLIN2, PPARG and ADIPOQ in C3H10T1/2 cells differentiated into adipocytes for seven days in the presence or absence of TGFβi 3µg/mL. B-C) Determination of gene expression by RT-PCR of the bone-related markers and inflammatory markers SPP1, RUNX2, CD44, GPNMB, AXIN2, TGFβi, IL6, VCAM and CCL2 in C3H10T1/2 cells differentiated into adipocytes for seven days in the presence of or absence of TGFβi 3µg/mL. D) Impact of TGFβi stimulation on C3H10T1/2 cell proliferation. Determination of cell proliferation based on counting the number of cells per well and RNA quantification in the differentiation of C3H10T1/2 to adipocytes for seven days in the presence or absence of TGFβi 3µg/mL.

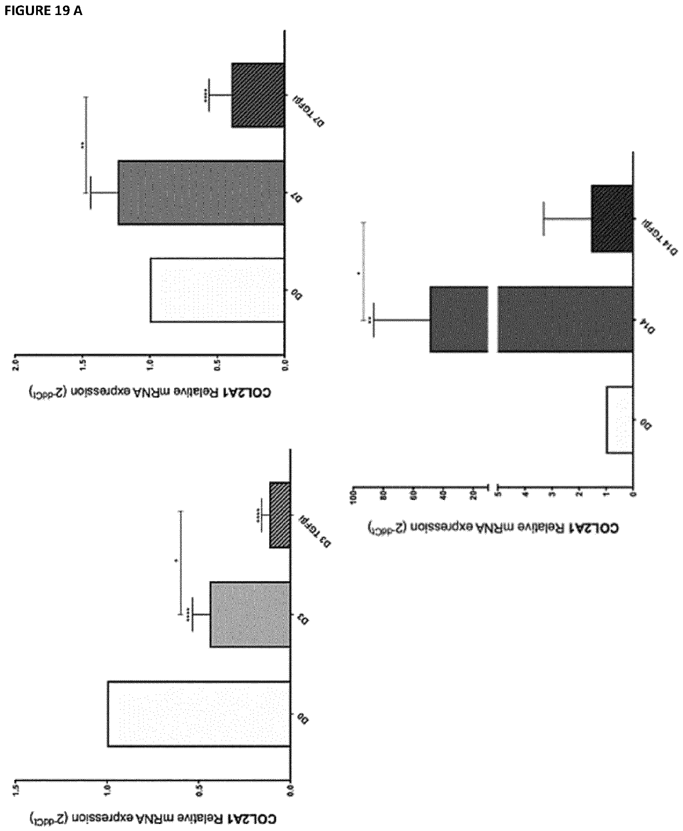

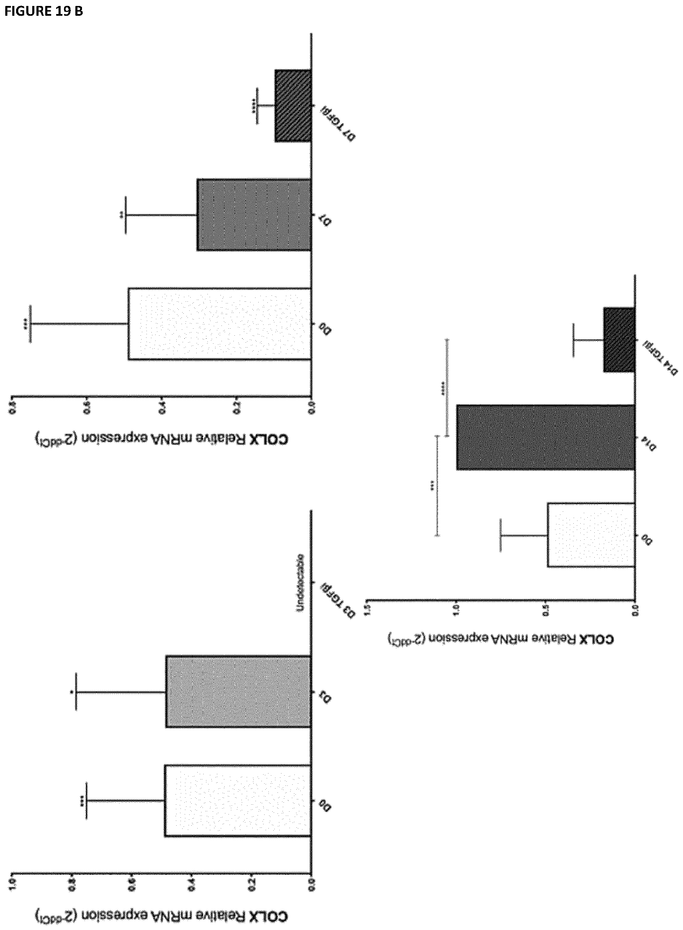

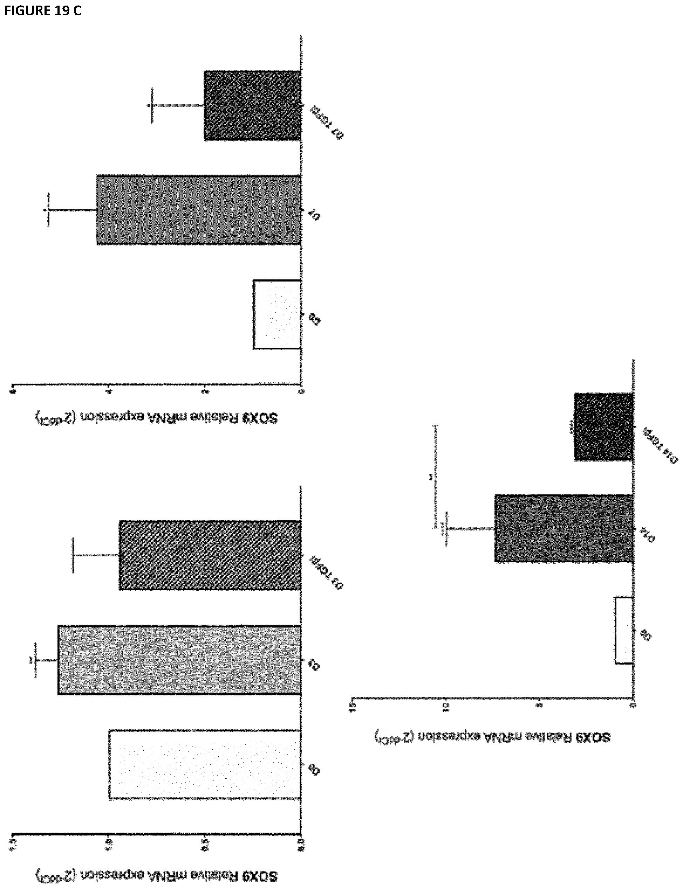

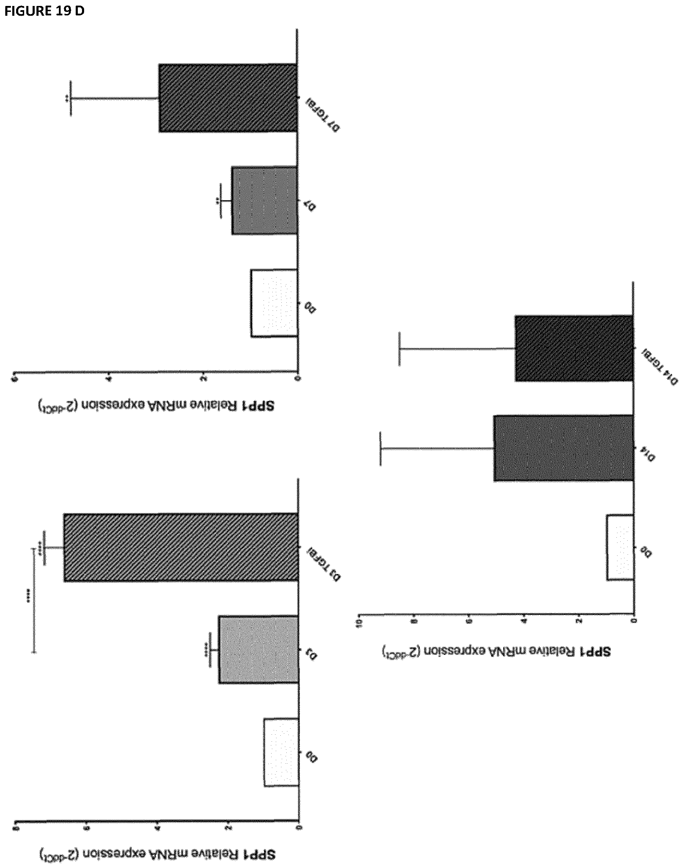

**Figure 19****. Effect of TGFβi on the chondrogenesis.** A-C) Gene expression measured by RT-PCR of the main cartilage components COL2A1, COLX, and the transcription factor SOX9 in ATDC5 cells differentiated into chondrocytes for three, seven, and fourteen days in the presence or absence of TGFβi 3µg/ mL. D) Expression of the bone marker SPP1 quantified by RT-PCR in the chondrogenic differentiation of ATDC5 cells in the presence or absence of TGFβi 3µg/mL for three, seven, and fourteen days.

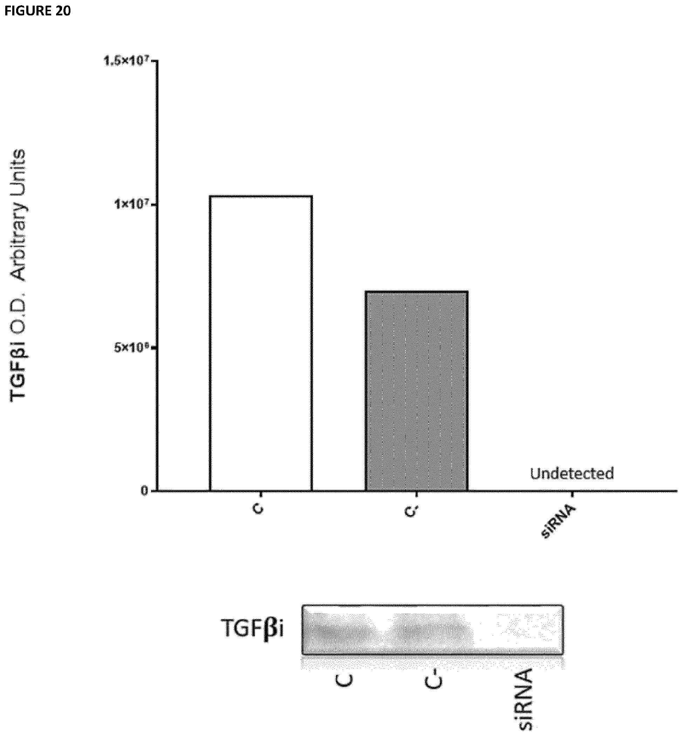

**Figure 20****. TGFβi blockade on astrocytes.** TGFβi protein expression after 48 hours of TGFβi silencing by using siRNA technique.

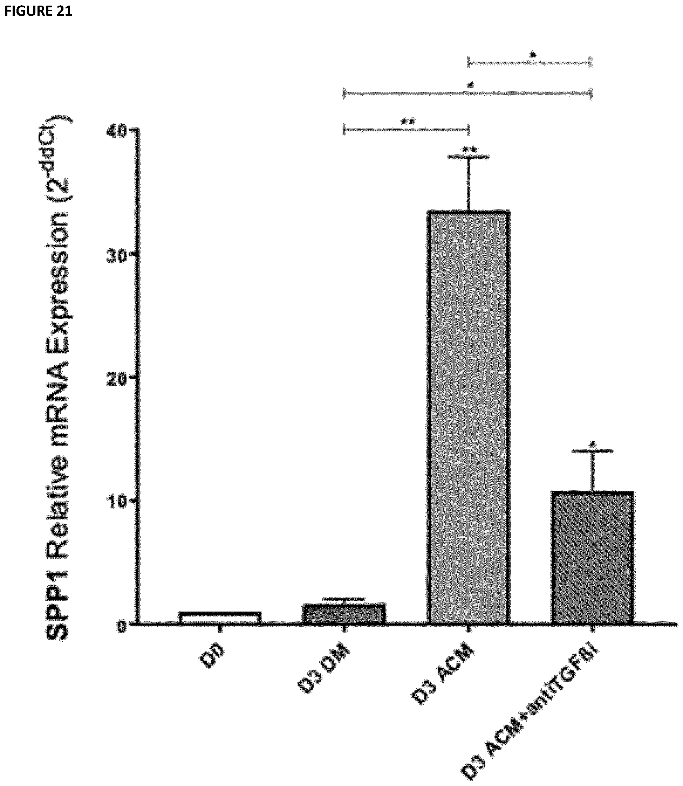

**Figure 21****. Effect of TGFβi blockade on ACM medium on osteoblasts.** SPP1 gene expression on SaoOS2 cells differentiated into osteoblasts after TGFβi blockade by adding a TGFβi antibody to the ACM during 1-hour previous SaOS2 stimulation.

### Detailed description of the invention

The present invention is illustrated by means of the Examples set below, without the intention of limiting its scope of protection.

### Example 1. Material and methods

### Example 1.1. Human Samples

### Blood Samples

Following the inclusion criteria **(Table 1),** blood samples were taken from 38 patients, and they were classified into 4 groups based on the risk of suffering NHO: group I (G1), patients with TBI and LBF at high risk of suffering HO; group II (G2), patients with TBI with a lower risk of suffering from the disease; group III (G3), patients with LBF with low risk of suffering from HO and group IV (G4), control patients with no risk of suffering from HO.

All blood samples were drawn after project approval by the Santiago and Lugo Area Research Ethics Committee (reference code 2017/262). Likewise, the collection of the samples was carried out after the subsequent signing by the patients or relatives of the informed consent regarding the treatment of the samples and the purpose of the investigation, in accordance with the Declaration of Helsinki.

Blood samples were taken at the time of hospital admission (D0), after 24h (D1), 72h (D3), and 168h post-trauma (D7), with the exception of G4, who only underwent one extraction

**Table 1.Inclusion Criteria**

| **Groups/Characteristics** | | | **G1** | **G2** | **G3** | **G4** |
|---|---|---|---|---|---|---|
| ***Polytraumatism*** | *TBI* | *NISS* | >16 | >16 | - | - |
| | | *Glasgow* | <13 | <13 | - | - |
| | *LBF* | | Yes | - | Yes | - |
| ***Age*** | | | 18-65 | 18-65 | 18-65 | 18-65 |

### Nerve Samples

Peripheral sciatic, posterior tibial, and saphenous nerves were obtained from human donors. The Research Ethics Committee of the Santiago and Lugo Area approved this procedure (registration code: 2017/262). Likewise, the samples were extracted after the pertinent signing by the relatives of the donors of the informed consent regarding the treatment of the samples and the purpose of the investigation, in accordance with the declaration of Helsinki.

### Example 1.2. Animal model

### Animals

Eighty-five male Sprague-Dawley rats (276.12 ± 15.2g) were obtained from Monash Research Platform (Clayton, Australia) were 7-weeks old. All rats were housed individually on a 12-hour light/dark cycle (lights on at 0700) with access to food and water ad libitum for the duration of the experiment. All procedures were approved by the Alfred Animal Ethics Committee (E/1923/2019/B) and were within the guidelines of the Australian Code of Practice for the Care and Use of Animals for Scientific Purposes by the Australian National Health and Medical Research Council.

### Experimental groups

Rats were assigned to one of four injury groups: polytrauma (POLY; muscle crush + fracture + TBI; n=32); peripheral injuries-only (PERI; muscle crush + fracture + sham-TBI; n=18); TBI-only (TBI; sham-muscle crush + sham-fracture + TBI; n=22); and sham (SHAM; sham-muscle crush + sham-fracture + sham-TBI; n=13). 18 POLY, 5 PERI, and 11 TBI rats were excluded due to either death immediately post-injury, euthanasia during the acute recovery period, or a comminuted fracture. This left a total of 14 POLY rats, 13 PERI rats, 11 TBI rats, and 13 SHAM rats.

### Injury methods

Extracranial injuries were administered as previously described (24). Briefly, anesthesia was given using 5% isoflurane in 2L/min oxygen, and subsequently maintained at 2% isoflurane (flow rate 500mL/min). Buprenorphine (dosage: 0.05 mg/kg) in sterile saline was administered subcutaneously before the muscle crush injury was performed. A 1.2 kg impactor (diameter: 1 cm, depth: 1.5 cm) was released from a height of 55 cm and guided by 2 metal rods to impact the right hamstring. Following the muscle injury, the femoral fracture was performed. First, an incision was made medial to the patella and the patella moved laterally to expose the femoral bone. A 1.1mm thick Kirschner wire was inserted into the marrow cavity to stabilize the fracture for the duration of the experiment. The patella was moved back to its original position (i.e., in front of the Kirshner wire) and stabilized by suture to facilitate locomotion. A 500 g impactor (diameter: 3 mm) was released from a height of 55 cm and guided to strike the femoral bone midshaft to induce a transverse non-comminuted femoral fracture which was confirmed via x-ray.

A TBI was administered following the extracranial injuries using the lateral fluid percussion injury (FPI) model. As previously described (25), following craniotomy (5 mm in diameter, 4.5 mm posterior, 2.5 mm left of bregma) a hollow injury cap was attached over the craniotomy using dental acrylic. The rat was then connected to the fluid percussion device (Model FP 302, Amscien Instruments, USA) by the injury cap, and a fluid pulse (~3 atmospheres) was delivered. For all sham injuries, anesthesia and buprenorphine were given before incisions and craniotomies were made and sutured, however, the weight was not released and FPI was not delivered.

### Blood collection

Under isoflurane-induced anesthesia, blood was collected 2 days after injury via the lateral tail vein using a 23G needle, into 500µl K2-EDTA microtainers (#365975, McFarlane Medical). Immediately, after collection, microtainer was then inverted gently to allow the blood and EDTA to mix prior to centrifugation at 1300g for 10 minutes at 4°C. 100uL supernatant (plasma) was aliquoted into each 0.5 mL Protein LoBind Tubes (#0030108434, Eppendorf) and stored at -80°C.

### Example 1.3. Cell cultures

### Cell culture

Human SaOS2 cells, and CCF-STTG1 astrocytes were obtained from CLS (CLS, Eppelheim, Germany). C3H10T1/2 MSCs were gently donated by Dra Pardo of IDIS Institute of Santiago de Compostela. ATDC5 cells were purchased from Riken Cell Bank.

### Cell differentiation

SaOS2 were differentiated for 3 days, C3H10T1/2 for 7 days, and ATDC5 for 14 days. In order to induce cell differentiation, the media were changed three times a week in SaOS2 and ATDC5 cells, starting the process 24h post-culture. In these cells, the same medium was used in all the changes made. Regarding the C3H10T1/2, two media were used to differentiate them into adipocytes: the induction medium which was changed after 6h post-culture, and the maintenance medium which replaced the induction medium after 4 days. after culture. All cells were seeded in 24-well plates (Thermo Fisher Scientific, Waltham, MA, USA).

On the other hand, SaOS2 cells were differentiated for 0, 3, 7, 14, and 21 days in the presence of 10% FBS or 10% serum from the patients of the four groups (G1, G2, G3, and G4). To do this, 58,000 cells/well were seeded in a 24-well plate. The next day the seed medium was removed and SaOS2 differentiation medium (DM) devoid of FBS was added. 10% FBS or 10% patients' serum was then added, and cell differentiation was performed as we previously described .

### Co-culture

Cell co-cultures were performed in Millicell^{®} hanging cell culture inserts (Thermo Fisher Scientific, Wlatham, MA, USA) with a pore size of 0.4 µm and 24-well plates. Astrocytes (CCF-STTG1) were cultured in the insert, at a density of 10,500 cells/insert. The following day, the medium of the astrocytes was changed to the DM and they were kept in culture for 72h. 48h after the seeding, the SaOS2 were seeded in the 24-well plates at a density of 58,000 cells/well. After 24h of the SaOS2 culture, both cultures were unified to form a coculture of CCF-STTG1 and SaOS2. At this time, the medium for both cells was changed to a new DM and they were kept in coculture for 3, 7, 14, and 21 days.

Regarding NE and SaOS2 cocultures, the same procedure described above for astrocytes was used. The only difference is that instead of the astrocytes, a NE was placed on the insert.

In the cocultures performed with the ONC, the protocol followed was exactly the same as that previously described for the NE. However, it differs from this in that the cells were seeded once confluence was reached in the well with the NE. For this, the ONC were seeded in the inserts with a density of 21,000 cells/insert. Subsequently, the SaOS2 were seeded to constitute the coculture of ONC and SaOS2, and it was maintained for 3 days.

### Example 1.4. Cell Cultures with the Conditioned Media

### Obtention of the Conditioned Media

Astrocyte Conditioned Medium (ACM): Astrocytes were seeded in 6-well plates at a density of 332,500 cells/well. After 24h post-culture, the medium was changed and 2mL of DM was added. Under these conditions, the astrocytes conditioned the medium for 72h to obtain the ACM.

Nerve Conditioned Medium (NCM): After processing the primary nerve sample, the NE were plated in a 6-well plate at a density of 3-4 ne/well. After a month and a half, and 72h after the conditioning of the medium (prior to its change), the NCM was collected.

### Cell Cultures with the Conditioned Media

Once the MCA was obtained, it was used to stimulate the differentiations of SaOS2 and C3H10T1/T2, as previously described.

After 24h SaOS2 post-culture, the medium was changed to ACM, and they were differentiated for 3 days.

Regarding the C3H10T1/2, due to the difference in the composition of their differentiation media and that of the SaOS2, we proceeded with the concentration and purification of the 2mL DM and ACM proteins, using the filter units 3kDa Amicon^{®} Ultra - 0.5mL Centrifugal Filters Ultracel 3K (Millipore, Burlington, Massachusetts, USA) following the supplier's instructions. Once the process was finished, the pellets were resuspended in the differentiation medium corresponding to the cell line, filtered through a 0.2µm filter (Thermo Fisher Scientific, Wlatham, MA, USA), and cells were differentiated for 7 days as was previously described.

For cell differentiation stimulated with the NCM, NCM was concentrated using Amicon^{®} Ultra - 0.5mL Centrifugal Filters Ultracel 3K 3kDa centrifugal filter units, according to the supplier's specifications. Then, they were resuspended with the C3H10T1/2 differentiation medium and filtered through a 0.2µm filter. Finally, C3H10T1/2 cells were differentiated into adipocytes in the presence and absence of NCM for 7 days, as previously described.

### Example 1.5. Treatments

### Inflammatory stimuli

To determine the effect of an external inflammatory factor on the differentiation process of SaOS2 stimulated with ACM, SaOS2 cells were seeded and stimulated with the ACM in the presence and absence of 0.1 ng/mL of IL1β and 100 ng/ mL of lipopolysaccharide (LPS). To do this, once the SaOS2 completed the 3-day differentiation with the ACM, these cells were treated with the inflammatory stimuli and were differentiated for a further 48h.

### Transforming Growth Factor Beta Induced (TGFβi) stimulation

SaOS2, C3H10T1/2, and ATDC5 cells were differentiated in the presence or absence of 3µg/mL of human recombinant protein transforming growth factor beta-induced (TGFβi), similar concentration to TGFβi-blood levels. It should be noted that this protein was added newly in every medium change corresponding to the differentiation of each cell line.

### Example 1.6. Cell viability

### RNA Quantification and Cell Counting

The RNA of the differentiated C3H10T1/2 was quantified using a NanoDrop One^{®} spectrophotometer (Thermo Fisher Scientific, Wlatham, MA, USA).

Moreover, microscopic photos of the different conditions were taken. Subsequently, the photograph was divided into 3 rectangles of equal size and positioned in the same place in the images. The live cells were counted, and the number of cells was compared with the rest of the samples.

### MTT Assay

Viability was tested using the MTT reagent. Briefly, 6 × 10³ cells/well were plated in 96-well plates and treated as described above. Then, the cells were incubated for 4h with MTT reagent. After formazan salt was dissolved, absorbance was measured at 570nm in a spectrophotometer.

### Example 1.7. Cell Staining

### Actin, tubulin and nucleus staining

SaOS2 cells were seeded on coverslips in a 24-well plate at a density of 58,000 cells/well. These cells were stimulated with the ACM and differentiated into osteoblasts for 3 days. After the differentiation period, the medium was removed from the well, and staining was performed following the supplier's instructions. Finally, the cells were visualized under a confocal microscope and photographs were taken.

### Oil Red O Staining

C3H10T1/2 cells were differentiated as we previously described. After completing 7 days of differentiation, cells were fixed with 4% formaldehyde at room temperature for 10 minutes. After this time, the formaldehyde was removed, and the cells were dried completely. Next, cells were incubated with 21% oil red O for 10 minutes and, subsequently, four washes with distilled water were carried out to eliminate the remains of non-specific staining. Finally, 500µL of distilled water were added, and photographs were taken under a microscope.

### Example 1.8. Western Blot

Supernatants and serum were precipitated using methanol and chloroform. After protein precipitation, pellets were resuspended in Ripa Buffer. Immunoblots were performed and visualized with Immobilon^{®} Western (Millipore, Burlington, Massachusetts, EEUU) using ChemiDoc MP Imaging System (BioRad Laboratories, Hercules, CA, USA). Data obtained were further validated by densitometric analysis using Image Lab^{™} Software (Image Lab^{™} 6.0.1, Bio-Rad Laboratories, EEUU) e ImageJ (ImageJ 1.51s, NIH, EEUU).

### Example 1.9. TGFβi Enzyme-linked immunosorbent assay (ELISA)

Plasma levels of TGFβi were quantified using Rat beta IG-H3/TGFBI ELISA Kit PicoKine^{®} (#EK1571, Boster Biological Technology, Pleasanton CA, USA). All samples and standards were run in duplicates and the assay was conducted as per the manufacturer's instructions.

Once the reaction is complete, the absorbance of each well was measured using FLUOstar Omega (BMG Lab Technologies) at 450 nm wavelength and processed with MARS Data Analysis software (BMG Lab Technologies) to calculate the mean protein concentration for each sample.

### Example 1.10. RNA extraction and real-time PCR (RT-PCR)

RNA was extracted using TriReagent^{®} and the RNA extraction Kit E.Z.N.A. Total RNA Kit I^{®} (Omega Bio-Tek Inc., Norcross, GA, USA) following the manufacturer's instructions. The gene expression of bone related markers, inflammatory and adipogenic markers were measured using iTaq Universal SYBR Green Supermix (BioRad Laboratories, Hercules, CA, USA). Relative quantitation was performed using the ΔΔCt Comparative Method. Results are expressed as 2^{-ΔΔCt} versus unstimulated control or DM.

### Example 1.11. Statistical Analysis

The obtained results from this work were expressed as the mean ± the standard deviation of the mean (SEM) of at least three experiments or independent samples and were analyzed using the GraphPad Prism statistical program (GraphPad Software Inc. 8, USA) and R (RStudio 1.3.1093, USA). For this analysis, the parametric and non-parametric Student's t test were used, according to the origin of the samples. A p value less than 0.05 (p<0.05) was considered significant. In the graphical representation, the results were expressed as * for p<0.05, ** for p<0.01, *** for p<0.001 and **** for p<0.0001. For the correlations, Pearson's non-parametric correlation analysis was used, applying 5% of the FDR.

### Example 2. Results

### Example 2.1. Effect of Astrocytes on Osteoblastogenesis

### Example 2.1.1. Study of the Anabolic Effect of Astrocytes on Osteoblasts

In the context of TBI, astrocytes play an important role in pathophysiological and repair processes through the release of numerous factors. In order to study the potential contribution of these cells in NHO, astrocytes (CCF-STTG1) and osteoblasts (SaOS2) were co-cultured and differentiated into osteoblasts for three, seven, fourteen, and twenty-one days. Interestingly, cell co-culture promoted osteoblastogenesis by increasing gene expression of essential markers of bone anabolism such as SPP1, RUNX2, and BMP2 in osteoblasts **(**Figure 1A-C).

To determine if this effect was due to the direct effect of the astrocytes on the osteoblasts, or if it was a result of the feedback between both cell types, astrocytes conditioned the DM for three days to obtain the ACM. Subsequently, osteoblasts were stimulated with ACM and differentiated for three days. The anabolic effects previously observed in cell co-culture were preserved, with the exception of the effect on RUNX2, which was not modified by the presence of ACM **(****Figure 2A****),** suggesting the release of anabolic factors by astrocytes. Based on these findings, we studied a greater number of bone metabolism markers. The obtained results evidenced that ACM increased the gene expression of the bone anabolism markers BMP4, LIF, GPNMB, CD44, PDGFβ , and its receptor PDGFRβ **(****Figure 2B****),** as well as OPG **(****Figure 2C****)** in osteoblasts. However, ACM did not induce changes in RANKL expression, which resulted in a reduction in the RANKL/OPG bone remodeling ratio **(****Figure 2C****).**

Finally, ACM modulated the main gene markers of the WNT pathway. Thus, ACM increased the gene expression of AXIN2 in osteoblasts, an inducible gene after the activation of the WNT pathway, which in turn acts as its inhibitor through a negative feedback mechanism; and decreased the gene expression of SOST and DKK1, the main inhibitors of this pathway **(****Figure 2C****).**

### Example 2.1.2. Determination of the inflammatory effect of the astrocyte conditioned medium on osteoblasts

Considering the role of inflammation in bone anabolism, the effect of ACM on inflammatory processes in osteoblasts was investigated. ACM increased the gene expression of relevant inflammatory markers such as VCAM, a gene related to leukocyte recruitment and vascular adhesion associated with inflammation; and CCL2, a gene that promotes monocyte chemotaxis and macrophage polarization in inflammatory processes, in SaOS2 differentiated into osteoblasts for three days (**Figure 3A**)**.**

In order to study the contribution of the intrinsic inflammatory effect of ACM to the inflammatory responses associated with innate immune responses that have been described in NHO, SaOS2 cells were stimulated with IL1β (0.1 ng/mL) and LPS (100 ng/mL) for 48h, after being differentiated into osteoblasts in the presence or absence of ACM for three days. The presence of ACM potentiated the inflammatory effect of IL1β, which was evidenced by increased gene expression of IL6, VCAM and CCL2 (**Figure 3B**)**.** Interestingly, the inflammatory stimulus increased the expression of bone anabolic genes BMP2 and LIF induced by ACM (**Figure 3B**)**.** However, IL1β did not modify the expression of SPP1 (**Figure 3B**)**.** Regarding LPS, the main Toll-like receptor 4 (TLR4) agonist, it did not alter the inflammatory profile or bone metabolism (**Figure 3C**)**.**

### Example 2.1.3. Determination of cellular morphological changes induced by the astrocyte conditioned medium

The results obtained demonstrated the capacity of astrocytes to increase the expression of bone related markers in SaOS2 cells. To determine the effect of ACM on osteoblast morphology, tubulin, actin, and cell nucleus staining were performed. The data revealed that ACM induced osteocyte-like morphological changes in SaOS2 **(****Figure 4A****).** Taking these findings into account, osteocyte markers in SaOS2 cells were studied by RT-PCR. Consistent with the staining results, ACM increased podoplanin (PDPN) gene expression (**Figure 4B**)**,** an early marker of osteoblast to osteocyte differentiation. However, ACM did not modify BGLAP, the other osteocyte marker studied (**Figure 4B**)**.**

### Example 2.2. Effect of Astrocyte Conditioned Medium on Adipogenesis

Previous results pointed to ACM as a promoter of bone anabolism in osteoblasts. Considering that osteoblasts and adipocytes share the same cellular origin, the MSCs, and that osteoblastogenesis and adipogenesis are balanced and antithetical processes, experiments were performed to test the effect of ACM on C3H10T1/2 differentiation to adipocyte for 7 days. The data obtained revealed a decrease in the gene expression of the adipogenic markers FABP4, PPARG, and ADIPOQ in cells treated with ACM. However, ACM did not alter the expression of PLIN2 (**Figure 5A**)**.**

In order to validate the obtained data, Oil Red O staining on C3H10T1/2 differentiated into adipocytes for 7 days in the presence or absence of ACM was performed. In concordance with the gene expression results, the obtained data revealed a decrease of fat deposits in cells treated with the DM, and complete inhibition of lipid droplets in cells stimulated with ACM, corroborating its anti-adipogenic effect (**Figure 5B**)**.**

Taking into account the capacity of MSCs to differentiate into both bone and fat, and since ACM inhibited adipogenesis, bone metabolism markers and inflammatory markers were studied in C3H10T1/2 differentiated into adipocytes for seven days. Interestingly, ACM was able to increase the gene expression of the markers SPP1, RUNX2, CD44, AXIN2 (**Figure 5C**), IL6, and VCAM (**Figure 5D**)**.** In contrast, this medium reduced GPNMB expression (**Figure 5C**) and did not modify CCL2 expression (**Figure 5D**).

Finally, during the differentiation process of these cells, variations in cell proliferation were observed. To determine these changes, an MTT assay of the C3H10T1/2 differentiated into adipocytes was performed for seven days in the presence and absence of the ACM. The obtained results showed an increase in cell proliferation of C3H10T1/2 stimulated with the ACM during their differentiation into adipocytes (**Figure 5E**).

### Example 2.3. Effect of Nerve Explants on Osteoblastogenesis

HO is a multifactorial disease whose etiology remains unknown. However, there are certain risk factors that increase its prevalence. Among them, neurological damage, such as a TBI and spine cord injury should be highlighted. Recently, studies have indicated the possible involvement of the PNS in the development of NHO. In order to investigate the role of peripheral nerves in ectopic bone formation, NE and SaOS2 were co-cultured and differentiated into osteoblasts for three days. The obtained data demonstrated an increase in the gene expression of the bone markers SPP1, RUNX2, BMP2, LIF, CD44, RANKL, the RANKL/OPG ratio, as well as an increase in the expression of the inflammatory markers VCAM and CCL2 in the cocultured SaOS2 with the NE (Figure 6A-D). On the contrary, the coexistence of NE and SaOS2 decreased the expression levels of BMP4 and GPNMB (**Figure 6B**), and did not modify the expression of PDGFβ, PDGFRβ, OPG, and AXIN2 (Figure 6B-C).

Likewise, ONC and SaOS2 were cocultured and differentiated into osteoblasts for three days. Despite the fact that the observed effects were not as strong as those observed in the cocultures of NE and SaOS2, ONC were able to increase SPP1 and VCAM gene expression in SaOS2 cells. Likewise, the presence of ONC increased the RANKL/OPG ratio and decreased the expression of BMP4 and PDGFRβ. However, this coculture did not induce changes in the other genes studied (**Figure 7**)**.**

Considering the inflammatory and anabolic effects observed in the coexistence of NE and SaOS2, the effect of combining the co-cultures of NE and SaOS2 stimulated with the ACM was investigated. The results showed how the presence of NE significantly increased the effects of the ACM in terms of gene expression of the osteoblastic and inflammatory markers SPP1, BMP2, LIF, RANKL, and CCL2 (Figure 8A-D). Interestingly, ACM synergized with the effects of the NE-osteoblast co-culture by increasing the gene expression of SPP1, LIF and CCL2 (**Figure 8A****,B,D**). On the contrary, NE significantly reduced the gene expression levels of BMP4, GPNMB, CD44, PDGFβ, PDGFRβ, and AXIN2 (Figure 8B-C). Likewise, ACM, beyond the previously mentioned synergistic induction, was able to modulate the effects of NE by significantly enhancing the expression of the markers BMP4, GPNMB, CD44, PDGFβ, OPG, and AXIN2 (Figure 8B-C)**;** and reducing the expression of the RUNX2, RANKL genes and the RANKL/OPG ratio **(**Figure 8A,C).

### Example 2.4. Effect of Nerve Explants Conditioned Medium on Adipogenesis

Considering the balance between osteoblastogenesis and adipogenesis, the effect of NCM was tested in C3H10T1/2 cells differentiated into adipocytes for seven days, and the main adipogenic markers were studied by RT-PCR. The results showed a significant decrease in the gene expression of three of the markers studied, FABP4, PPARG, and ADIPOQ. However, NCM increased the expression of PLIN2 (**Figure 9A**)**.**

Similarly, bone metabolism and inflammatory markers were studied in the adipogenesis differentiation model. The data obtained revealed a significant increase in the expression of SPP1, CD44, IL6, VCAM, and CCL2 in the cells stimulated with the NCM (Figure 9B-C)**.** However, this medium did not modify the expression of RUNX2, GPNMB and AXIN2 (**Figure 9B**)**.**

### Example 2.5. Characterization of the Astrocyte Conditioned Medium and Nerve Explant Conditioned Medium

Given the results obtained, we characterized the secretome of the astrocytes, the NE, as well as the DM by proteomics.

The data obtained from the qualitative proteomic analysis (DDA) evidenced the elevated expression of TGFβi in the ACM compared to the DM (**Figure 10A**). These results were confirmed by the proteomic quantitative analysis (SWATH). Thus, SWATH analysis revealed the increase of this protein in the ACM, which increased 14.52 more in the ACM than in the MD (Figure 10A-B). In order to validate the proteomic data, protein expression studies of cell supernatants were performed using western blot. The obtained information showed a higher protein expression of TGFβi in the ACM compared to the DM, which was in concordance with the previous results obtained by proteomics (**Figure 10C**).

Likewise, we characterized the NCM as we did with the ACM. The obtained results in the qualitative proteomic analysis (DDA) showed a greater expression of TGFβi in the NCM in relation to the DM (**Figure 11A**)**.** Likewise, the quantitative proteomic study (SWATH) confirmed this increase, which was 2.2 times higher in the NCM compared to the DM **(**Figure 11A-B). These results were validated by western blot, which revealed an increase in the protein expression of TGFβi in the NCM when compared to the NM (**Figure 11C**).

Finally, ACM and NCM proteomic analyses were compared. The qualitative analysis revealed two common proteins in both media: TGFβi, and heavy chain inter-alpha-trypsin inhibitor 4 (ITIH4). Interestingly, TGFβi was the only common protein that remained significant in the ACM and NCM quantitative proteomic analyses.

In view of these results, the expression levels of TGFβi in SaOS2 differentiation to osteblasts, and C3H10T1/2 to adipocyte in presence or absence of the ACM, NE, and ONC were analyzed by RT-PCR. The obtained results evidenced the capacity of ACM to increase the gene expression of TGFβi in osteoblastogenesis (**Figure 12**) and in adipogenesis (**Figure 13**)**.** However, the increase in TGFβi in the adipogenic model was not significant when compared to the control. Similarly, the presence of NE and ONC increased TGFβi gene expression in both osteoblastic (**Figure 12**) and adipogenic (**Figure 13**) differentiation. Likewise, the expression of this gene was studied in the SaOS2 differentiated in the presence of the coculture with the NE and the ACM. The results obtained showed how ACM was able to significantly enhance the expression of TGFβi in relation to NE; and how NE significantly reduced the expression of this gene in cells treated with the ACM (**Figure 12**).

### Example 2.6. Identification of TGFβi Expression in NHO in vivo model

In order to validate the previous results, we studied TGFβi expression in blood samples collected from the rats of the NHO in vivo model. Results elucidated a significant increase of this protein in the double trauma group (TBI+LBF) when compared to the rest of the groups (**Figure 14A**)**.** Moreover, TGFβi was higher increased in the animals which developed the ectopic bone (including animals from TBI+LBF and LBF groups) when compared to the rest of the groups (**Figure 14B**)**.**

### Example 2.7. Identification of TGFβi Expression in Patients at Risk of Suffering from NHO

In order to characterize the patients with a higher or lower risk of suffering from NHO, serum samples from G1, G2 and G3 patients were analyzed by proteomics. This data was correlated with the changes in the gene expression induced by the patient's sera during SaOS2 differentiation. The results demonstrated a greater number of correlations with bone metabolism in those patients who presented the double trauma (G1) in relation to the rest of the groups (G2 and G3) (Figure 15A-C)**.** In addition, the proteome enrichment revealed a greater relationship with the inflammatory cascades and pathways that involve TGFβ signaling in patients at high risk of suffering from NHO (G1). Altogether, this data correlates with the results obtained in vitro (**Figure 15D**)**.**

Since *in vitro* and *in vivo* experiments identified TGFβi as a potential contributor to the anabolic and inflammatory processes of NHO, and in view of the differences in the proteome of the different groups of patients, kinetics studies of TGFβi expression in serum were performed. The results revealed an increase in this protein in patients at high risk of developing NHO (G1), reaching its maximum expression on the third day after the trauma (**Figure 16**)**.**

### Example 2.8. Validation of TGFβi as a Potential Protein Involved in the Formation of Ectopic Bone

### Example 2.8.1. Effect of TGFβi Stimulation on Osteoblastogenesis

We previously demonstrated the pro-osteoblastogenic and anti-adipogenic effects of ACM and NCM. Likewise, these studies pointed to TGFβi as a potential biomarker of NHO due to its high expression induced by both ACM and NCM, as well as its presence in the rat's serum and in the serum of patients at risk of suffering from this disease. In order to elucidate the involvement of TGFβi in this process, SaOS2 cells were differentiated in the presence or absence of the human recombinant protein TGFβi 3µg/mL for three days. Interestingly, treatment with TGFβi partially reproduced the effects of ACM on osteoblasts. Thus, TGFβi increased the gene expression of SPP1, BMP2, TGFβi, VCAM and CCL2 (**Figure 17**). On the contrary, stimulation with TGFβi did not modify the gene expression of the other genes studied (**Figure 17**).

### Example 2.8.2. Effect of TGFβi Stimulation on Adipogenesis

C3H10T1/2 cells were differentiated into adipocytes for seven days in the presence or absence of TGFβi 3µg/mL. Treatment with the recombinant protein reduced the gene expression levels of all adipogenesis markers studied, including FABP4, PLIN2, PPARG and ADIPOQ (**Figure 18A**)**.** However, PPARG was the only marker whose reduction was statistically significant (**Figure 18A**)**.**

In the same way, the bone and inflammatory related markers were also studied in C3H10T1/2 MSCs differentiated into adipocytes. The results revealed an increase in the gene expression of SPP1, RUNX2, CD44, TGFβi, and VCAM in C3H10T1/2 stimulated with TGFβi (Figure 18B-C). On the contrary, no modifications were observed in the gene expression of the rest of the genes studied **(**Figure 18B-C).

Finally, the stimulation of the cells with the recombinant protein TGFβi promoted cell proliferation, which was determined by the increase in the number of cells, and the amount of RNA in the cells stimulated with it **(****Figure 18D**).

### Example 2.8.3. Effect of TGFβi Stimulation on Chondrogenesis

Chondrocytes play an essential role in bone formation through the endochondral ossification process. Therefore, the effect of treatment with the recombinant protein TGFβi 3µg/mL on ATDC5 differentiation into chondrocytes for three, seven, and fourteen days was evaluated. The obtained data reflected a decrease in the gene expression of the COL2A1, COLX and SOX9 markers, evidencing the deleterious effect of TGFβi on chondrogenic differentiation **(**Figure 19A-C). In addition, TGFβi stimulation increased SPP1 expression in early stages of ATDC5 differentiation **(****Figure 19D**).

### Example 2.9. TGFβi Blockade

In this work, it is determined that TGFβi is a potential therapeutic target for NHO. Thus, as possible examples of TGFβi inhibitors, two different approaches to inhibit its actions were explored. First, a specific siRNA was used to silence the mRNA of TGFβi in astrocytes. Results showed that this approach is an effective way to blunt TGFβi released by astrocytes to the cell culture medium (**Figure 20**). Second, a neutralizing TGFβi antibody was added to the ACM to block TGFβi actions. As data revealed, SaOS2 cells differentiated to osteoblasts for 3 days in the presence of this antibody expressed lower mRNA levels of SPP1, a gene related to NHO (**Figure 21**).

## Claims

1. *. In vitro* method for the diagnosis or prognosis of Neurogenic Heterotopic Ossification which comprises assessing the expression level of TGFBi in a biological sample obtained from the subject, wherein the determination of an increase of the expression level of TGFBi with respect to a pre-established expression level measured in healthy control subjects, is an indication that the subject may be suffering from Neurogenic Heterotopic Ossification or has a poor prognosis.

2. . *In vitro* method, according to claim 1, wherein the biological sample is selected from: blood, plasma, serum, peripheral nerves comprising saphenous, posterior tibial and sciatic nerves or synovial fluid.

3. . *In vitro* use of TGFBi for the diagnosis or prognosis of Neurogenic Heterotopic Ossification.

4. . *In vitro* use of a kit comprising reagents for the determination of the level of expression of TGFBi for the diagnosis or prognosis of Neurogenic Heterotopic Ossification.

5. . TGFBi inhibitors for use in the treatment of Neurogenic Heterotopic Ossification.

6. . TGFBi inhibitors for use, according to claim 5, selected from siRNA which is completely base-paired to the target TGFBi mRNA or TGFBi neutralizing antibodies.

7. . TGFBi inhibitors for use, according to any of the claims 5 or 6, wherein the method comprises silencing TGFBi in astrocytes by using a siRNA and/or sequestering TGFBi present in the astrocyte secretome by using a neutralizing antibody against TGFBi.

8. . TGFBi inhibitors for use, according to any of the claims 5 to 7, wherein the TGFBi inhibitor is administered intravenously, locally or intra-articularly.

9. . An *in vitro* method for identifying compounds for the treatment of Neurogenic Heterotopic Ossification, which comprises a) determining if the inhibition of TGFBi or the elimination of the anabolic effects of astrocytes on osteoblasts has taken place by the candidate compound, and b) wherein if said inhibition of TGFBi or the elimination of the anabolic effects of astrocytes on osteoblasts has taken place, it is indicative of the candidate may be effective in the treatment of Neurogenic Heterotopic Ossification.
